# EUROPEAN PATENT SPECIFICATION

(11) **EP 3 666 897 B1**
(45) Date of publication and mention of the grant of the patent: **03.09.2025**
(21) Application number: 18840774.6
(22) Date of filing: 31.07.2018
(51) Int. Cl.: C07K 1/107, C12N 15/62

(54) **SUPER VERSATILE METHOD FOR PRESENTING CYCLIC PEPTIDE ON PROTEIN STRUCTURE**
ÜBERAUS VIELSEITIGES VERFAHREN ZUR DARSTELLUNG EINES ZYKLISCHEN PEPTIDS AUF EINER PROTEINSTRUKTUR
PROCÉDÉ SUPER-POLYVALENT POUR PRÉSENTER UN PEPTIDE CYCLIQUE SUR UNE STRUCTURE PROTÉIQUE

(30) Priority: 31.07.2017 JP 2017148622
(43) Date of publication of application: 17.06.2020
(73) Proprietor: The University Of Tokyo, Tokyo 113-8654 (JP); Osaka University, Suita-shi, Osaka 565-0871 (JP)
(72) Inventor: SUGA, Hiroaki, Tokyo 113-8654 (JP); TAKAGI, Junichi, Suita-shi Osaka 565-0871 (JP)
(74) Representative: D Young & Co LLP
(86) International application number: PCT/JP2018/028705
(87) International publication number: WO 2019/026920

(56) References cited:
- EP-A1- 2 267 003
- WO-A2-2012/016245
- JP-A- 2002 526 108
- JP-A- 2003 501 041
- JP-A- 2008 514 201
- JP-A- 2013 046 637
- JP-A- 2015 042 159
- TUNJUNG MAHATMANTO: "Review seed biopharmaceutical cyclic peptides: From discovery to applications : Seed Biopharmaceutical Cyclic Peptides", BIOPOLYMERS, vol. 104, no. 6, 1 November 2015 (2015-11-01), US, pages 804 - 814, XP055706394, ISSN: 0006-3525, DOI: 10.1002/bip.22741
- KENICHIRO ITO ET AL: "Artificial human Met agonists based on macrocycle scaffolds", NATURE COMMUNICATIONS, vol. 6, 11 March 2015 (2015-03-11), pages 6373, XP055362332, DOI: 10.1038/ncomms7373

## Description

### Technical Field

The present invention relates to a method of presenting a cyclic peptide on a protein having a loop structure, and the like.

### Background Art

There is known a technology of modifying an amino acid residue of a protein or introducing another peptide structure into a protein with a view to providing a protein having improved activity.

Patent Document 1 discloses a chimeric polypeptide having improved bioactivity and containing a serum albumin protein having a bioactivity different peptide sequence inserted therein.

Patent Document 2 discloses that modifications to at least one loop region of an FnIII based polypeptide and also modifications to a β sheet of the FnIII based polypeptide result in an FnIII based binding molecule with improved binding ability for a target molecule.

Patent Document 3 discloses a molecule obtained by incorporating one or more bioactive peptides in the Fc domain of an antibody protein. More specifically, a bioactive peptide is inserted into amino acid residues adjacent to each other in the loop region of an Fc domain.

Patent Document 4 similar to Patent Document 3 describes a modified Fc molecule. Disclosed is an Fc molecule having an additional functionality portion covalently bound through a side chain of an amino acid residue at a conjugation site. More specifically, the amino acid residue at the conjugation site is a cysteine residue.

In addition, Patent Documents 5 and 6 disclose a technology of modifying at least one site of the structure loop of an immunoglobulin, thereby giving the immunoglobulin with novel bonding ability.

In Patent Document 2, a binding active species to a target is obtained from a library of randomized peptide sequences inserted into the loop structure portion of FnIII.

Also in Patent Documents 5 and 6, an active species having binding activity to a target is screened from a library obtained by not inserting an existing peptide but inserting a randomized peptide sequence into one site of a specific structure loop of an immunoglobulin.

This means that only a library having a randomized peptide inserted therein is obtained by the above-described conventional technologies and it is not known whether with regard to a peptide portion whose insertion is identified by screening the library, the binding activity to the target can be maintained by only the peptide itself or not. In other words, the inserted peptide has a sequence inserted for obtaining a library and is not expected to have binding ability. In addition, it is unknown whether the interchangeability of a protein can be maintained, for example, whether the original activity of a peptide presented by immunoglobulin can be maintained even after reinsertion into FnIII.

JP2002526108 describes the use of scaffold proteins, particularly green fluorescent protein (GFP), in fusion constructs with random and defined peptides and peptide libraries.

JP2003501041 describes novel chimeric Ad- vectors carrying transgene, or portions of transgenes for gene transfer into diverse cell types or tissues in a CAR- and/or αᵤβ_{3/5}- independent manner. Also described are methods for producing such vectors and the use thereof for gene therapy to target a specific cell type or tissue.

Tunjung Mahatmanto et al. ("Review seed biopharmaceutical cyclic peptides: From discovery to applications : Seed Biopharmaceutical Cyclic Peptides", Biopolymers, 104, 6, 2015, pp 804-814) provides a review of mini-proteins (or peptides) with disulfide bonds and cyclic backbones for applications in medicine due to their stable and amenable to grafting epitopes with desirable activities. Part of the review focuses on MCoTI-II and SFTI-I scaffolds with intercysteine loops. The loops are said to be amenable to mutation.

EP2267003 describes enzymatic methods for providing a proteinaceous substance comprising a polypeptide of interest and a cyclic affinity tag. The method involves providing a precursor proteinaceous substance and at least one motif of the general formula X1-Tag-X2, wherein X1 and X2 represent amino acids whose side chains can be linked enzymatically by a covalent bond and Tag is an amino acid sequence capable of binding to a binding partner when cyclized, contacting said precursor with at least one enzyme capable of forming a covalent bond between X1 and X2, and isolating the resulting cyclized proteinaceous substance.

### Prior Documents

### Patent Document

Patent Document 1: Japanese Translation of PCT Application No. 2005-505243
Patent Document 2: Japanese Translation of PCT Application No. 2013-539362
Patent Document 3: Japanese Translation of PCT Application No. 2008-514201
Patent Document 4: Japanese Translation of PCT Application No. 2009-504164
Patent Document 5: Japanese Translation of PCT Application No. 2009-541361
Patent Document 6: Japanese Translation of PCT Application No. 2009-540837

### Summary

### Technical Problem

An object of the present invention is to provide a method of presenting an amino acid sequence derived from a cyclic peptide on a scaffold protein having a loop structure while retaining the structure and activity which the cyclic peptide has.

### Solution to Problem

The present invention is as defined in the appended claims. The following are the details of the present invention.
(1) A method of presenting an amino acid sequence derived from a cyclic peptide, having a binding activity to an intended molecule, on a scaffold protein having a loop structure, the method comprising:
   using, as the cyclic peptide, a peptide having a chemically crosslinked structure for forming an intramolecular cyclic structure, and
   replacing the chemically crosslinked structure of the cyclic peptide with two amino acid residues from the loop structure of the scaffold protein and fusing the resulting amino acid sequence derived from the cyclic peptide to the scaffold protein having a loop structure while retaining the binding activity of the cyclic peptide to the intended molecule, wherein the two amino acid residues constituting the loop structure have a Cα atomic distance within a range of from 4 to 7 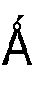 .
(3) The method described in (1), wherein the protein having a loop structure has from 1 to 15 amino acid residues between the two amino acid residues constituting the loop structure.
(4) The method described in any of (1) or (3), wherein the cyclic peptide is composed of a proteinogenic amino acid and/or a non-proteinogenic amino acid.
(5) The method described in any of (1) to (4), wherein the chemically crosslinked structure contains a thioether bond or a disulfide bond.
(6) The method described in any of (1) to (5), wherein two or more cyclic peptides each fuse to respectively different loop structures of a scaffold protein having a plurality of loop structures.
(7) The method described in (6), wherein the two or more cyclic peptides have the same amino acid sequence.
(8) The method described in (6), wherein the two or more cyclic peptides have respectively different amino acid sequences.
(9) The method described in any of (1) to (8), wherein in replacing the chemically crosslinked structure of the cyclic peptide with the two amino acid residues from the loop structure, the chemically crosslinked structure of the cyclic peptide is replaced, via a linker sequence, with the two amino acid residues constituting the loop structure.
(10) The method described in any of (1) to (9), wherein the chemically crosslinked structure of the cyclic peptide is replaced with the two amino acid residues from the loop structure while including an amino acid sequence constituting the loop structure other than the two amino acids from the loop structure.
(11) A method of fusing a partial amino acid sequence derived from a cyclic peptide, having a binding activity to an intended molecule, to a scaffold protein having a loop structure, thereby producing a modified protein having the partial amino acid sequence derived from the cyclic peptide presented on the protein, the method comprising:
   using, as the cyclic peptide, that having a chemically crosslinked structure for forming an intramolecular cyclic structure,
   selecting, from an amino acid sequence of the cyclic peptide, a partial amino acid sequence to be presented on the scaffold protein and selecting a nucleobase sequence corresponding to the partial amino acid sequence,
   selecting nucleobase sequences corresponding to two amino acid sequences from the loop structure of the scaffold protein having a loop structure, respectively, removing a base present between the selected nucleobase sequences if necessary, and preparing a nucleic acid having, inserted and incorporated therein, the nucleobase sequence corresponding to the partial amino acid sequence of the selected cyclic peptide, and
   translating the nucleic acid and wherein the resulting modified protein retains the binding activity to the intended molecule, and wherein the two amino acid residues constituting the loop structure have a Cα atomic distance within a range of from 4 to 7A.

### Advantage of the Invention

The present invention makes it possible to provide a method of presenting a cyclic peptide on a protein having a loop structure while retaining the structure and activity of the cyclic peptide with the protein as a scaffold.

### Brief Description of Drawings

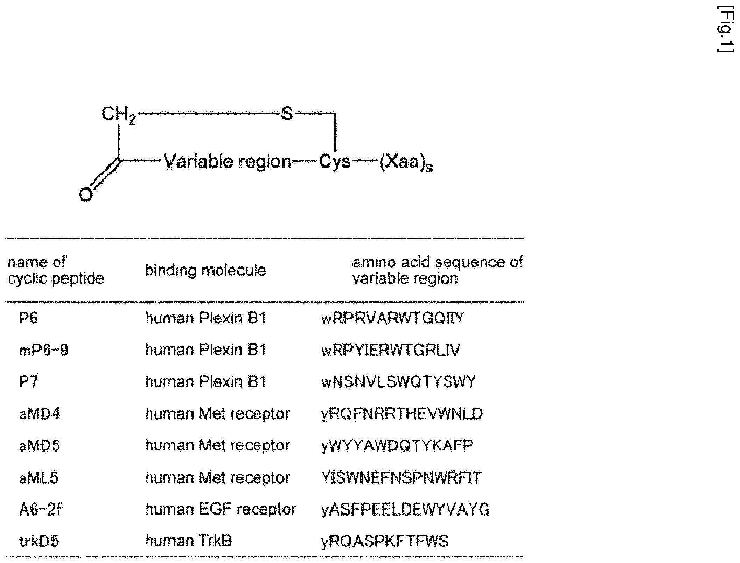

FIG. 1 shows the structure of cyclic peptides used in Examples. In the formula of the cyclic peptides, S represents a sulfur atom derived from the thiol group of Cys, Xaa represents an arbitrary amino acid, and s stands for an arbitrary integer of 0 or more. The variable region shows an amino acid sequence constituting the internal structure of the cyclic peptide by one-letter code and the amino acid represented by a small letter (w, y, or the like) is an amino acid in D form (D-Trp, D-Tyr).

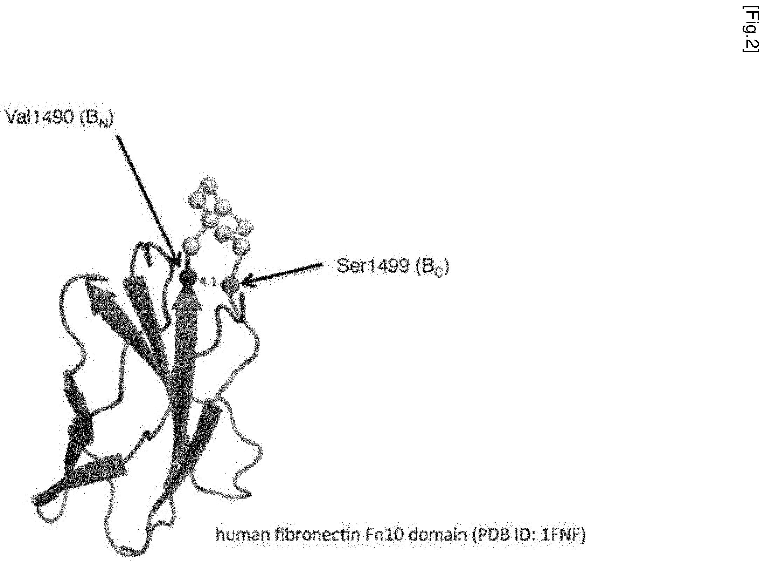

FIG. 2 shows the steric structure of a tenth Type III repeat domain of fibronectin used as a scaffold protein. The loop structure in the tenth Type III repeat domain is comprised of a loop structure of 8 amino acid residues between Val1490 as B_{N} and Ser1499 as B_{C}.

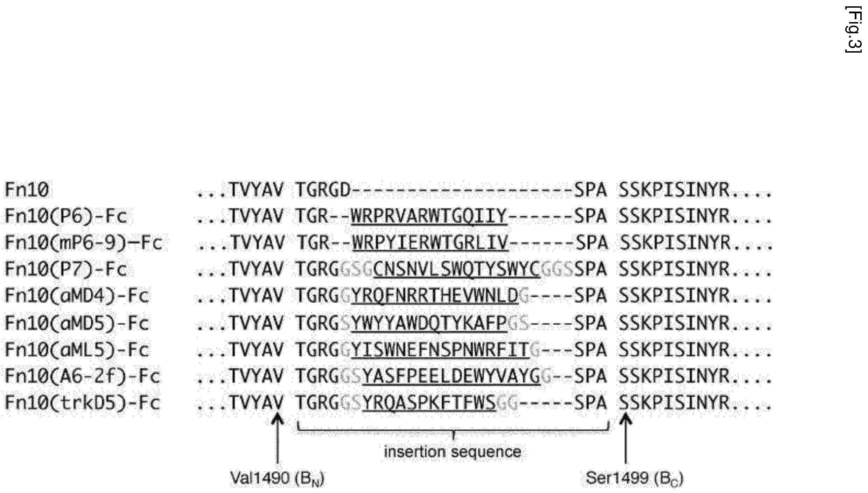

FIG. 3 shows an amino acid sequence of a loop portion after a cyclic peptide is fused, which is a partial structure of the fusion protein obtained using a tenth Type III repeat domain of human fibronectin as a scaffold protein. The sequence between Val1490 (B_{N}) and Ser1499 (B_{C}) is an insertion sequence. TGR and SPA in the insertion sequence is an amino acid sequence derived from the tenth Type III repeat domain of human fibronectin, the underlined portion is an amino acid sequence derived from the cyclic peptide, and the gray character stands for a linker sequence.

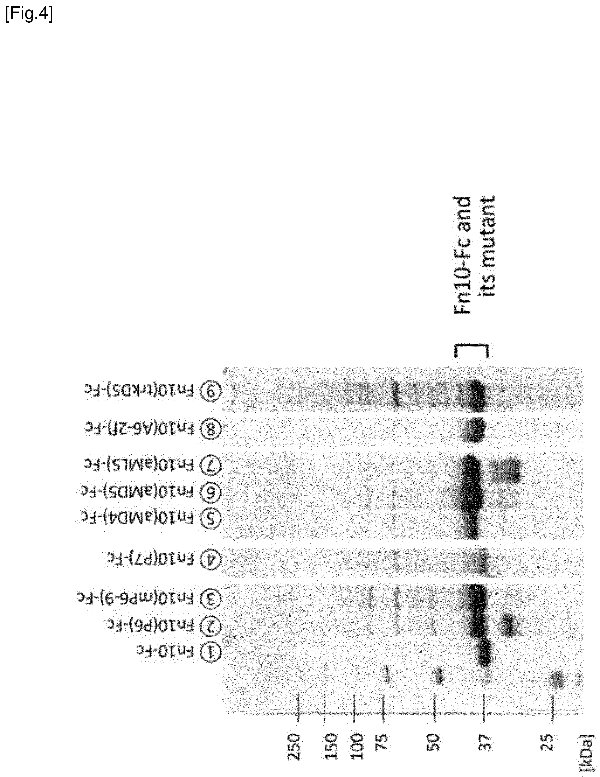

FIG. 4 shows SDS-PAGE of Fn10-Fc and cyclic peptide fusion proteins thereof expressed·secreted from Expi293F cells.

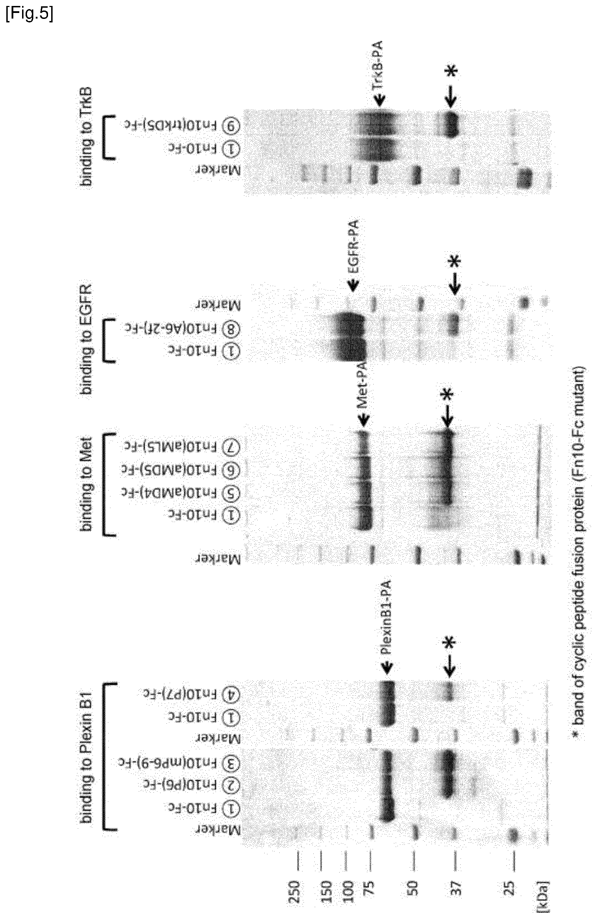

FIG. 5 shows the interaction results between the cyclic peptide fusion proteins (Fn10-Fc mutants) and binding molecules.

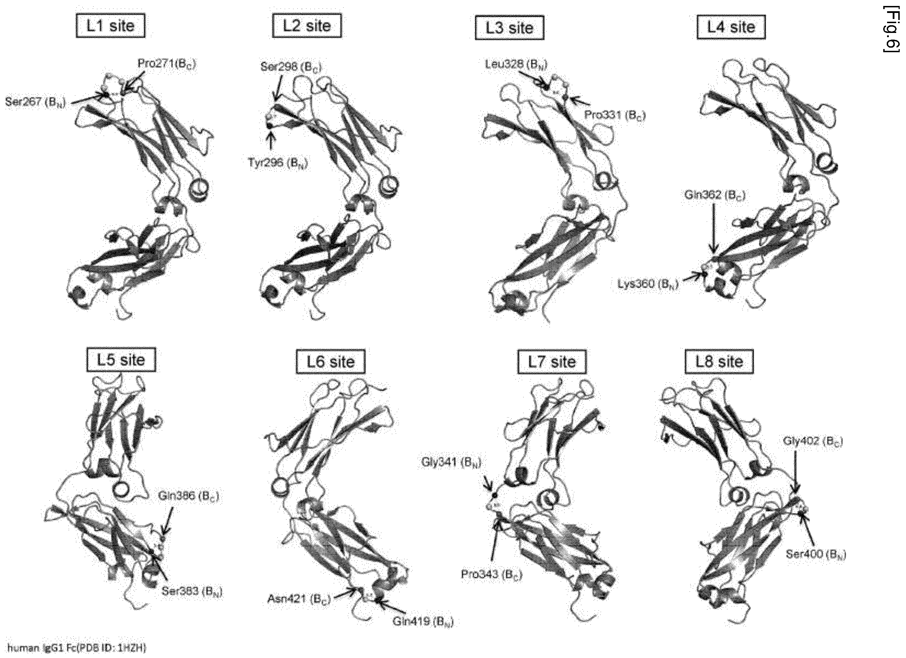

FIG. 6 shows the steric structure of a human IgG-derived Fc region used as a scaffold protein. The loop structures in the human IgG-derived Fc region are, as shown as L1 sites to L8 sites, respectively, comprised of, in each site, an amino acid residue represented by B_{N}, an amino acid residue represented by B_{C}, and a loop region of from 1 to 3 amino acid residues present therebetween.

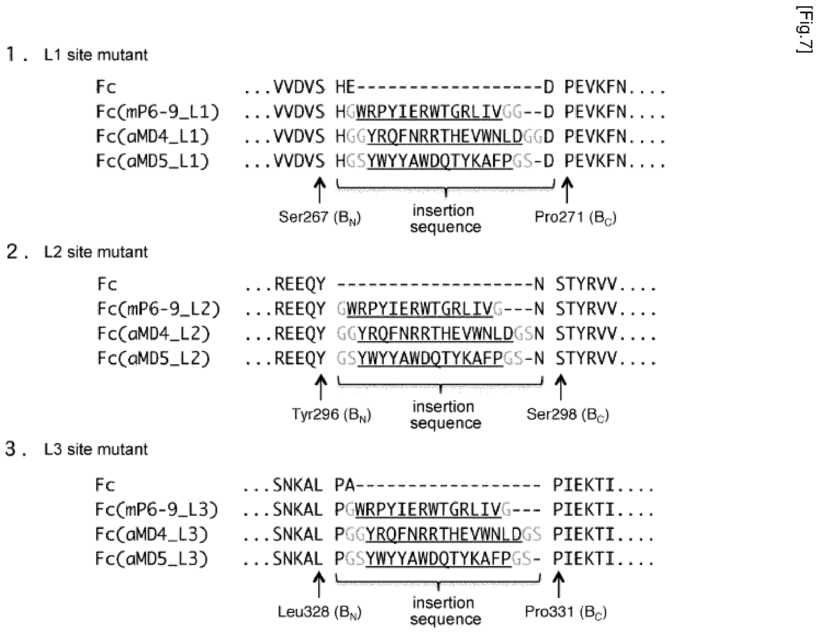

FIG. 7 shows an amino acid sequence of a loop portion after a cyclic peptide is fused, which is a partial structure of a fusion protein obtained using a human IgG-derived Fc region as a scaffold protein. Fusion proteins with a cyclic peptide in L1 to L3 sites at an "upper portion" near the hinge region of the Fc region are shown as L1 to L3 site mutants, respectively. The underlined portion is an amino acid sequence derived from the cyclic peptide and the gray character stands for a linker sequence.

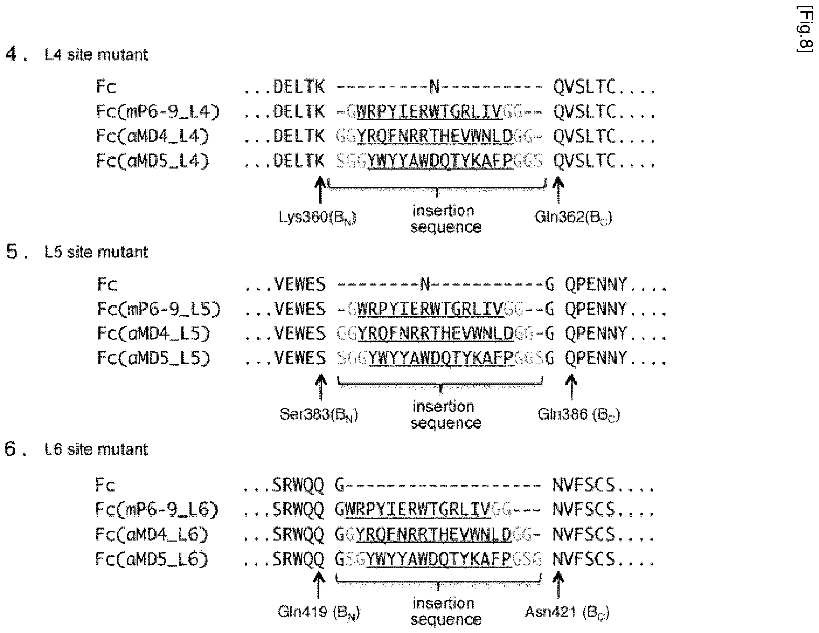

FIG. 8 shows an amino acid sequence of a loop portion after a cyclic peptide is fused, which is a partial structure of a fusion protein obtained using a human IgG-derived Fc region as a scaffold protein. Fusion proteins with a cyclic peptide in L4 to L6 sites at a "bottom portion" of the Fc region are shown as L4 to L6 site mutants, respectively. The underlined portion is an amino acid sequence derived from the cyclic peptide and the gray character stands for a linker sequence.

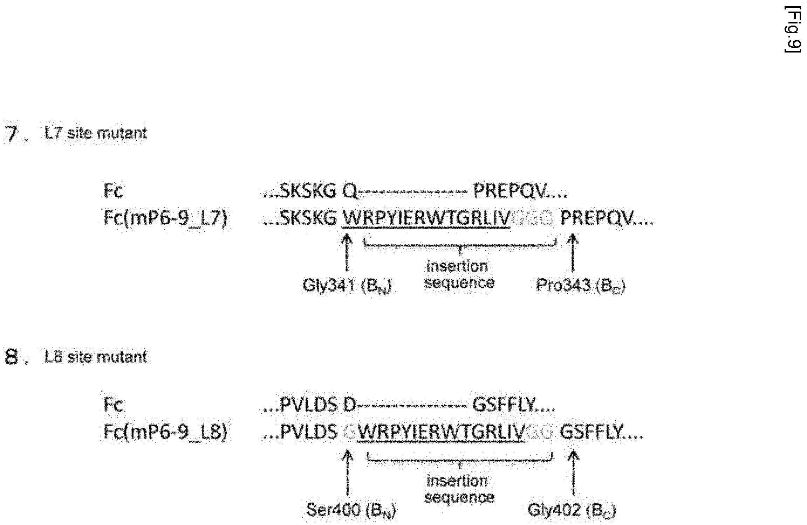

FIG. 9 shows an amino acid sequence of a loop portion after a cyclic peptide is fused, which is a partial structure of a fusion protein obtained using a human IgG-derived Fc region as a scaffold protein. Fusion proteins with a cyclic peptide in L7 and L8 sites at a "side-surface portion" of the Fc region are shown as L7 to L8 site mutants, respectively. The underlined portion is an amino acid sequence derived from the cyclic peptide and the gray character stands for a linker sequence.

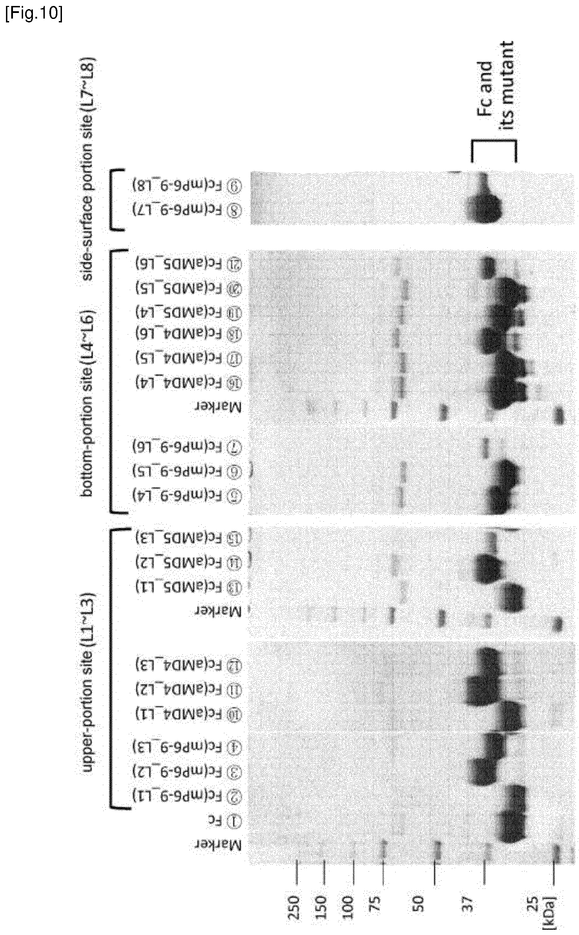

FIG. 10 shows SDS-PAGE of Fc and cyclic peptide fusion proteins thereof expressed·secreted from Expi293F cells.

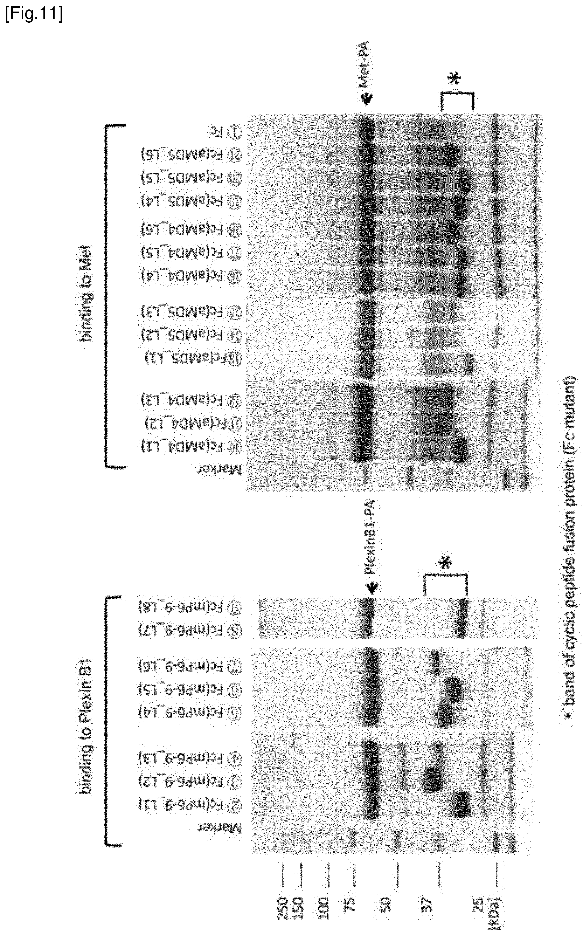

FIG. 11 shows the interaction results between the cyclic peptide fusion proteins (Fc mutants) and binding molecules.

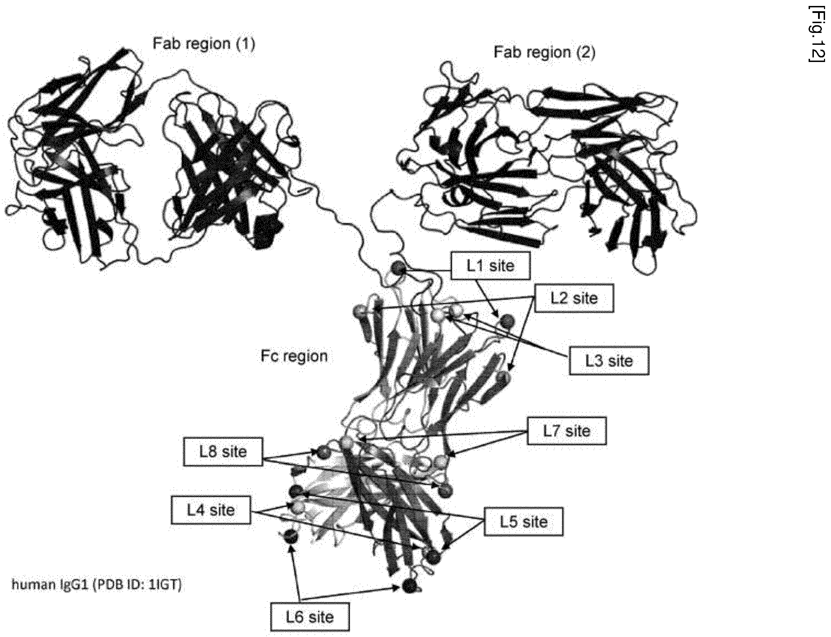

FIG. 12 shows the steric structure of the entirety of human IgG used as a scaffold protein. The loop structures in the Fc region used for fusion are shown as L1 site to L8 site in FIGS. 6 to 9 and here, their position is expressed as a midpoint of respective amino acid residues shown by B_{N} and B_{C} in FIG. 6.

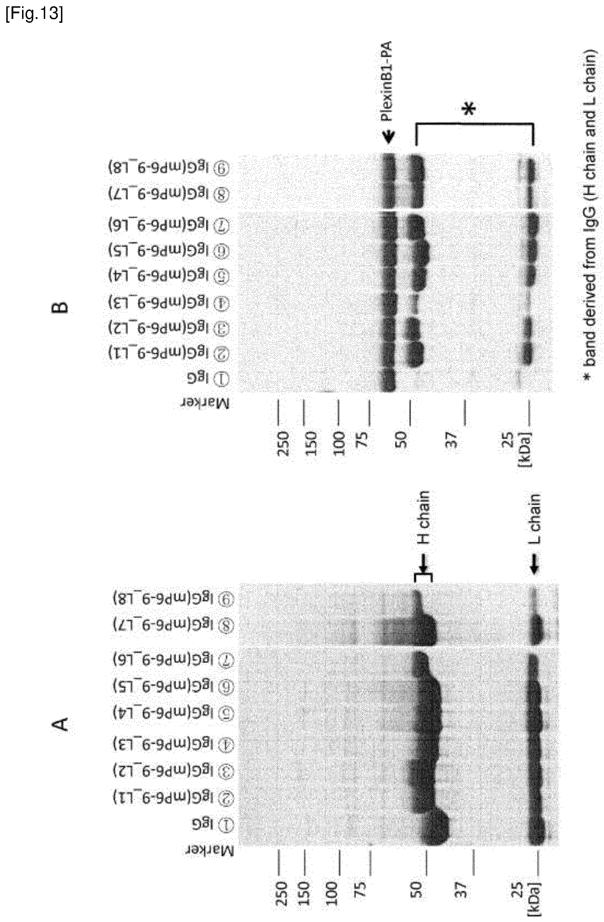

FIG. 13A shows SDS-PAGE of IgG and cyclic peptide fusion protein thereof expressed·secreted from Expi293F cells. FIG. 13B shows the interaction results between the cyclic peptide fusion proteins (IgG mutants) and a binding molecule.

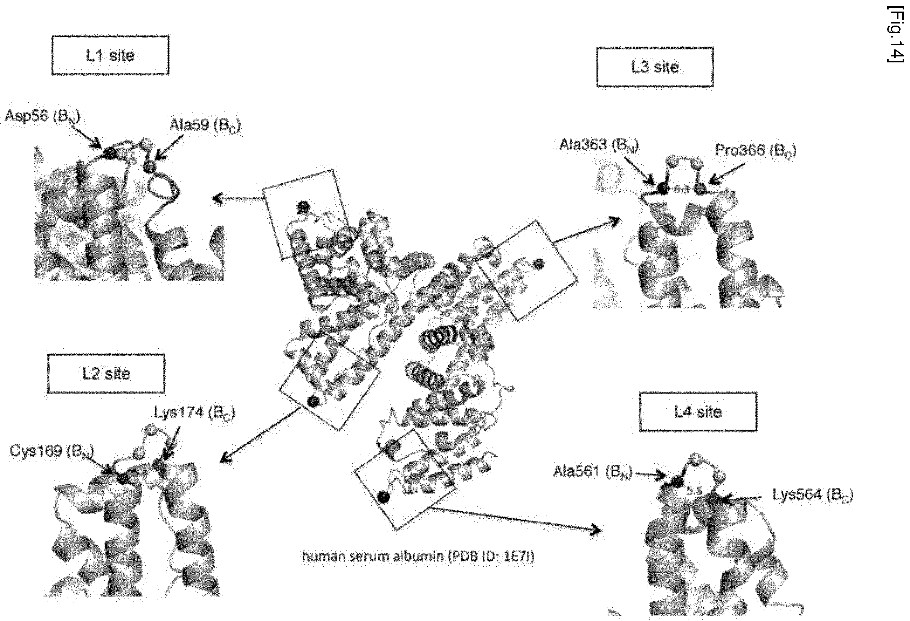

FIG. 14 shows the steric structure of human serum albumin used as a scaffold protein. The loop structures of the human serum albumin are, as shown by L1 to L4 sites, each comprised of, in each site, an amino acid residue represented by B_{N}, an amino acid residue represented by B_{C}, and a loop region of two or four amino acid residues present therebetween.

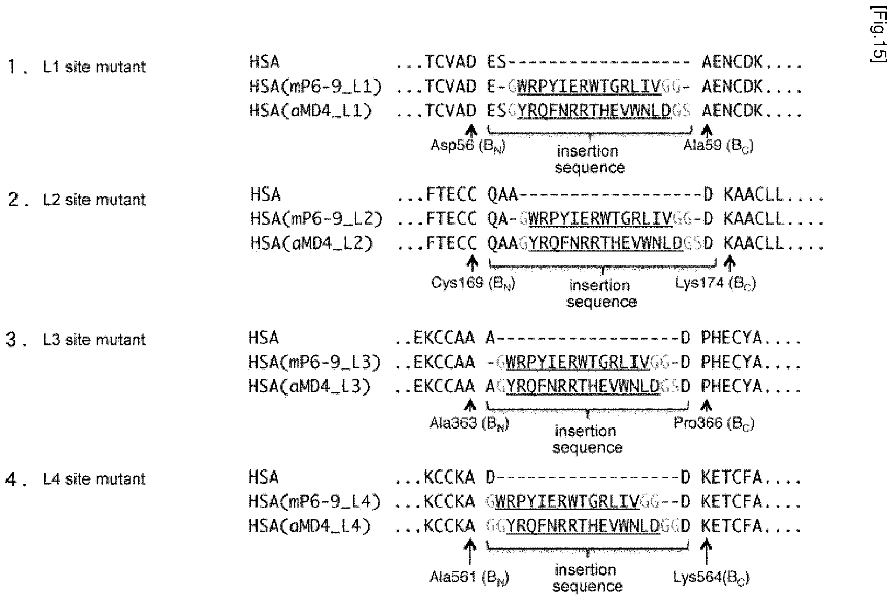

FIG. 15 shows an amino acid sequence of a loop portion after a cyclic peptide is fused, which is a partial structure of the fusion protein obtained using human serum albumin as a scaffold protein. The fusion proteins obtained by fusion with a cyclic peptide in L1 to L4 sites are shown as L1 to L4 site mutants, respectively. The underlined portion is an amino acid sequence derived from the cyclic peptide and the gray character stands for a linker sequence.

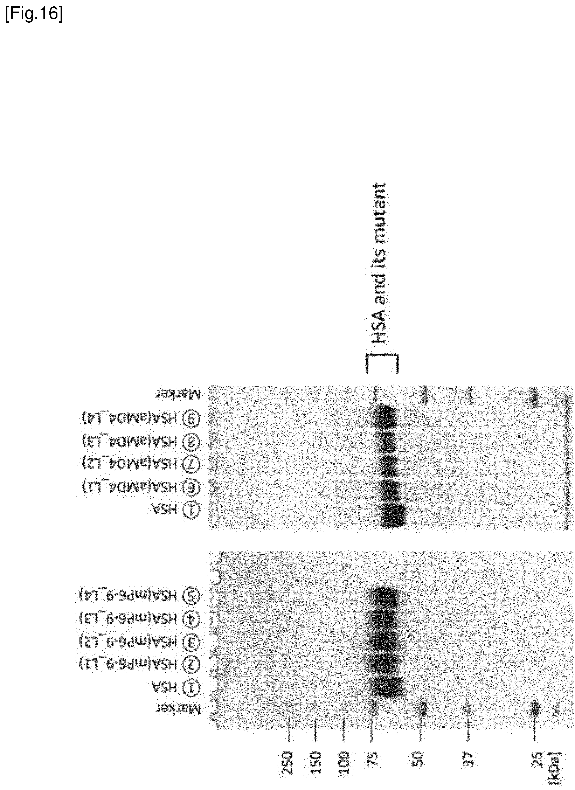

FIG. 16 shows SDS-PAGE of HSA and cyclic peptide fusion proteins thereof expressed·secreted from Expi293F cells.

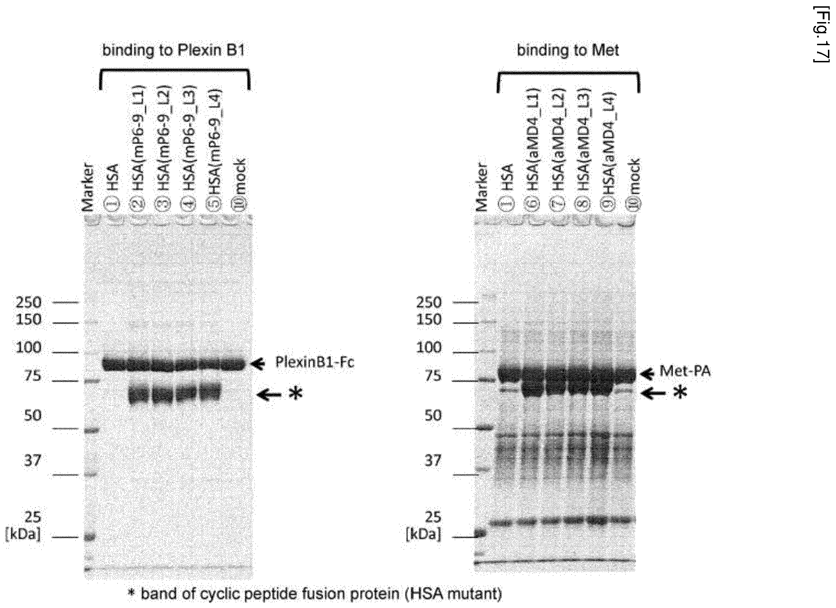

FIG. 17 shows the interaction results between the cyclic peptide fusion proteins (HSA mutants) and binding molecules.

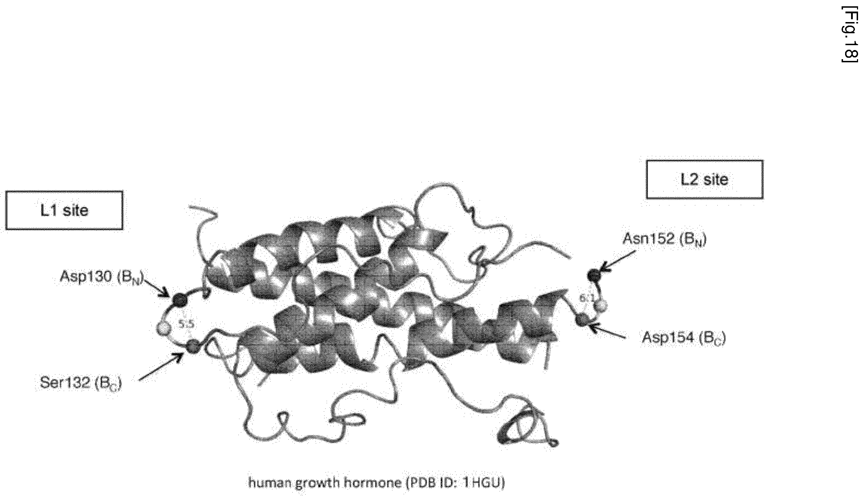

FIG. 18 shows the steric structure of human growth hormone used as a scaffold protein. The loop structures of the human growth hormone are, as shown as L1 and L2 sites, respectively, each comprised of, in each site, an amino acid residue represented by B_{N}, an amino acid residue represented by B_{C}, and a loop region of one amino acid residue present therebetween.

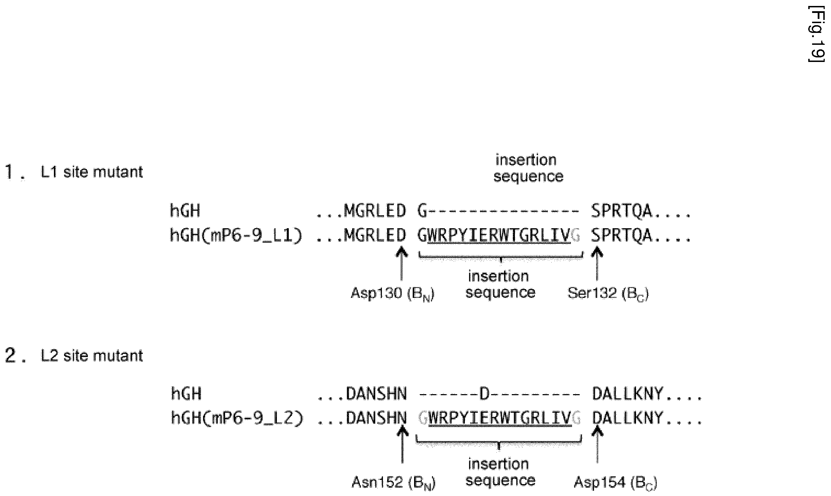

FIG. 19 shows an amino acid sequence of a loop portion after a cyclic peptide is fused, which is a partial structure of a fusion protein obtained using human growth hormone as a scaffold protein. The fusion proteins obtained using a cyclic peptide in L1 and L2 sites are shown as L1 and L2 site mutants, respectively. The underlined portion is an amino acid sequence derived from the cyclic peptide and the gray character stands for a linker sequence.

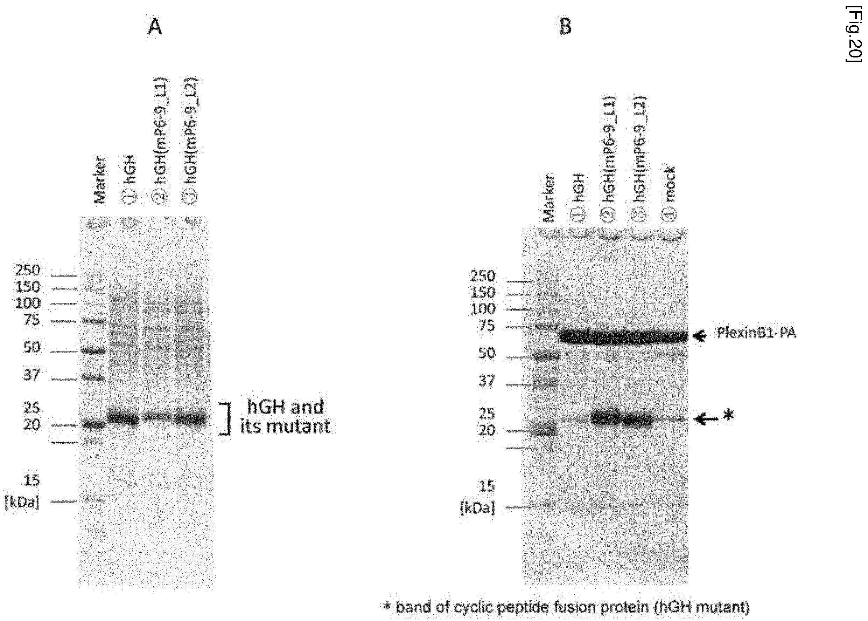

FIG. 20A shows SDS-PAGE of hGH and cyclic peptide fusion proteins thereof expressed·secreted from Expi293F cells. FIG. 20B shows interaction results between the cyclic peptide fusion proteins (hGH mutants) and a binding molecule.

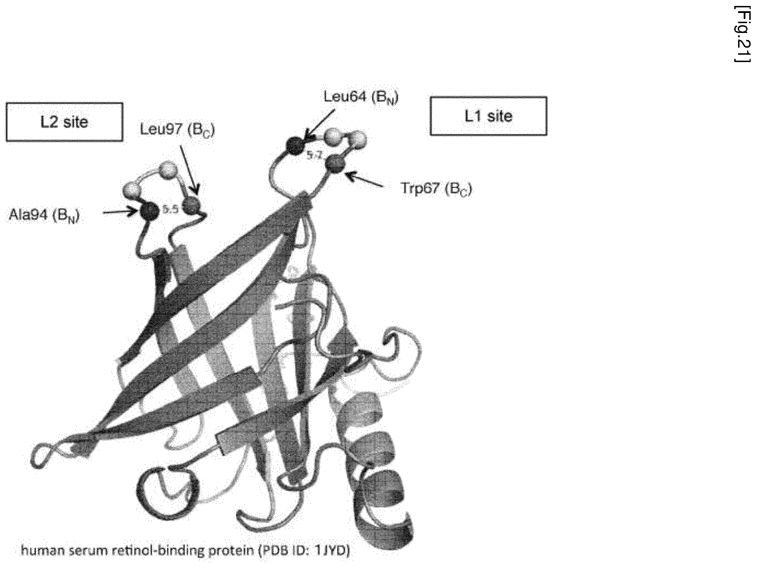

FIG. 21 shows the steric structure of a human serum retinol-binding protein used as a scaffold protein. The loop structures of the human serum retinol-binding protein are, as shown as L1 and L2 sites, respectively, comprised of, in each site, an amino acid residue represented as B_{N}, an amino acid residue represented by B_{C}, and a loop region of two amino acid residues present therebetween.

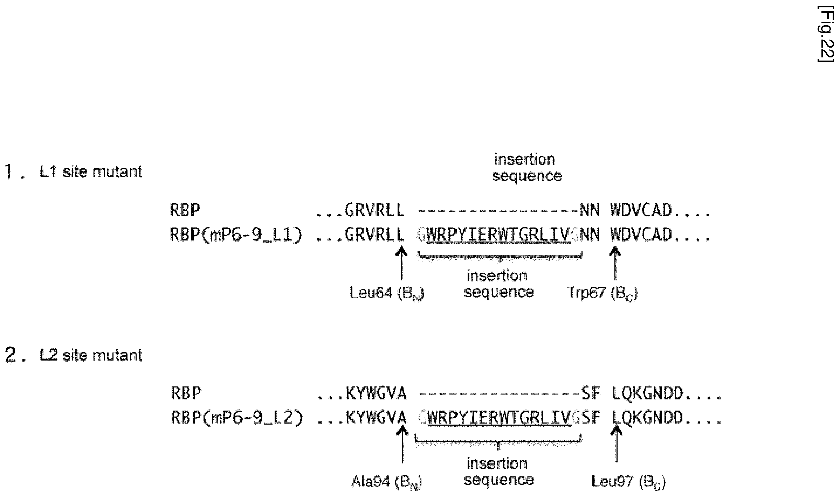

FIG. 22 shows an amino acid sequence of a loop portion after a cyclic peptide is fused, which is a partial structure of a fusion protein obtained using human serum retinol-binding protein as a scaffold protein. The amino acid sequence of a loop portion after fusion with the cyclic peptide is shown. The fusion proteins obtained using a cyclic peptide in L1 and L2 sites are shown as L1 and L2 site mutants, respectively. The underlined portion is an amino acid sequence derived from the cyclic peptide and a gray character stands for a linker sequence.

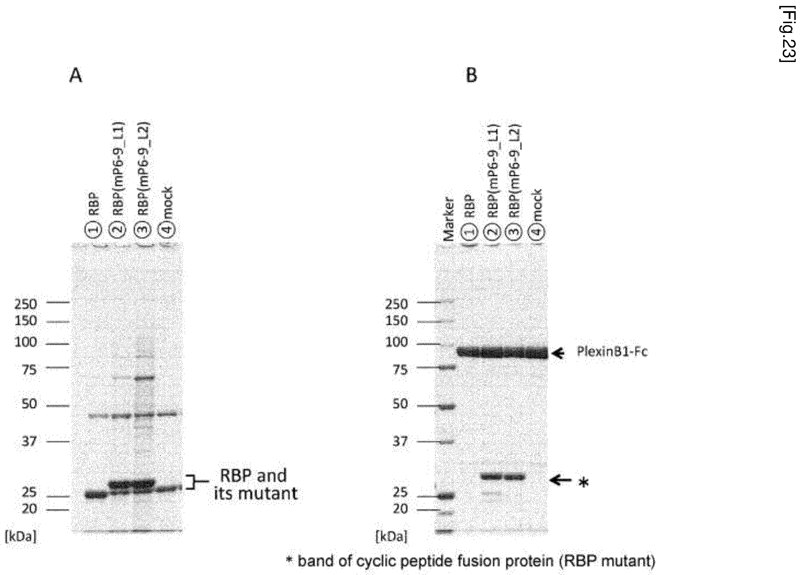

FIG. 23A shows SDS-PAGE of human serum retinol-binding protein (RBP) and cyclic peptide fusion proteins thereof expressed·secreted from Expi293F cells. FIG. 23B shows interaction results between the cyclic peptide fusion proteins (RBP mutants) and a binding molecule.

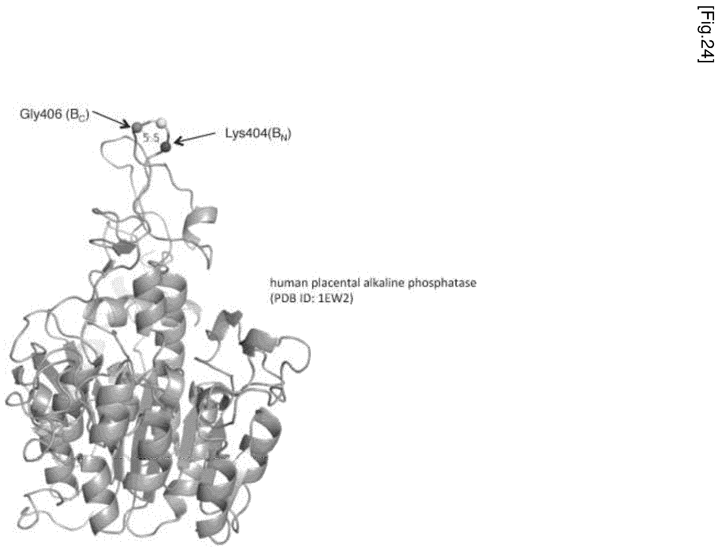

FIG. 24 shows the steric structure of human placental alkaline phosphatase used as a scaffold protein. The loop structure of the human placental alkaline phosphatase is comprised of, in each site, Lys402 as B_{N}, Gly404 as B_{C}, and a loop region of one amino acid residue therebetween.

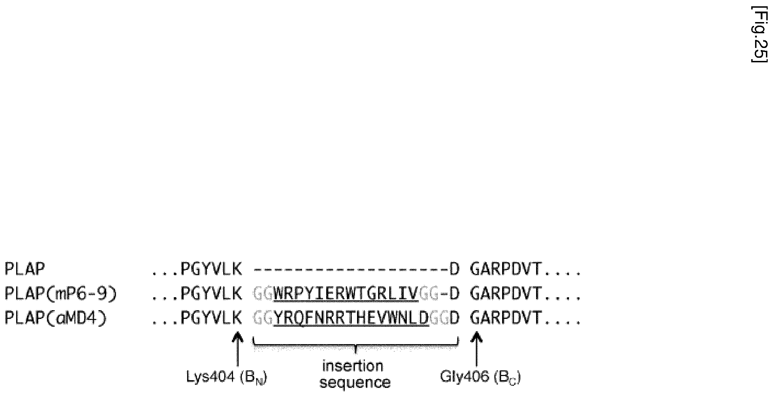

FIG. 25 shows the amino acid sequence of a loop portion after a cyclic peptide is fused, which is a partial structure of a fusion protein obtained using human placental alkaline phosphatase as a scaffold protein. The underlined portion is an amino acid sequence derived from the cyclic peptide and the gray character stands for a linker sequence.

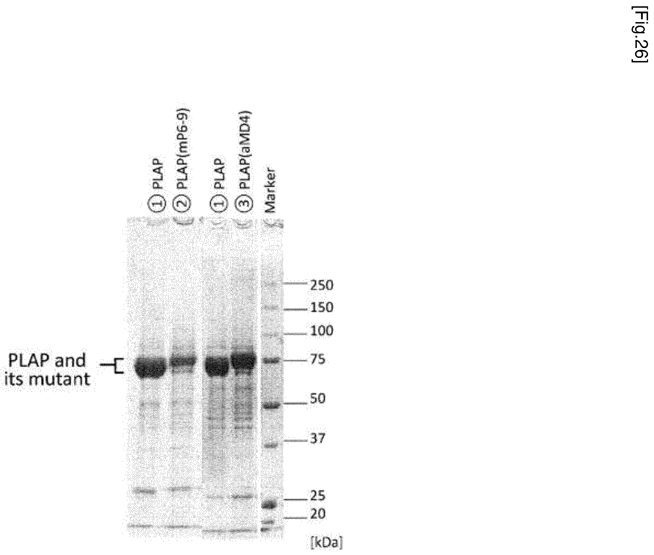

FIG. 26 shows SDS-PAGE of human placental alkaline phosphatase (PLAP) and cyclic peptide fusion proteins thereof expressed·secreted from Expi293F cells.

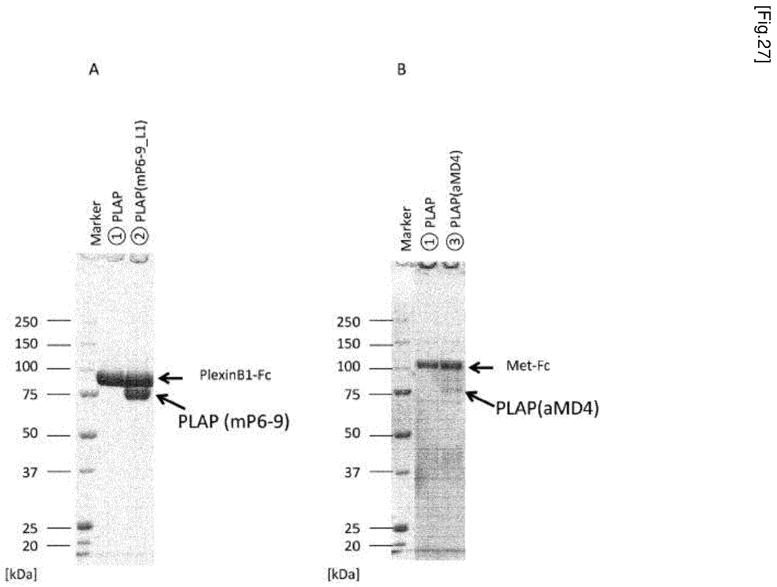

FIG. 27 shows interaction results between the cyclic peptide fusion proteins (PLAP mutants) and binding molecules.

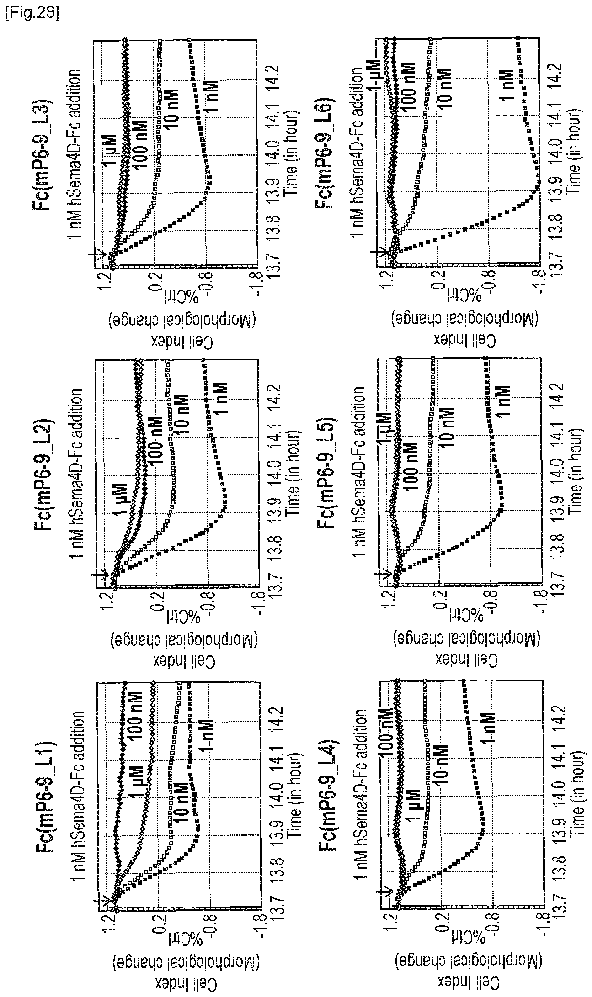

FIG. 28 shows an effect of mP6-9 peptide fusion proteins (Fc mutants) on inhibition of Plexin B1 signal transduction.

### Description of Embodiments

The present invention will hereinafter be described specifically by embodiments for carrying out the invention, but the present invention can be performed in various modifications without being limited by the following embodiments.

The present invention provides a method of presenting an amino acid sequence derived from a cyclic peptide on a protein having a loop structure. The cyclic peptide has a chemically crosslinked structure for forming an intramolecular cyclic structure and this method includes replacing the chemically crosslinked structure of the cyclic peptide with two amino acid residues from the loop structure, thereby fusing the cyclic peptide to the protein having a loop structure.

According to the present invention, an amino acid sequence derived from a cyclic peptide can be presented on a protein having a loop structure with the protein as a scaffold while maintaining the structure and activity of the cyclic peptide. The present invention has such advantages that a cyclic peptide presented on a protein with the protein as a scaffold can have increased bioadaptability and the function of the protein can be given to the cyclic peptide.

In the present invention, the amino acid sequence derived from a cyclic peptide is presented on the protein in such a manner as to replace its partial structure with the loop structure of the protein. Preferably, the partial structure of the cyclic peptide replaced with the loop structure is a structure for exhibiting activity, more specifically, physiological activity. From the structural viewpoint, the partial structure of the cyclic peptide to be replaced with the loop structure is preferably selected from amino acid sequences constituting the intramolecular cyclic structure of the cyclic peptide.

The present invention can provide a highly versatile method of presenting an amino acid sequence derived from a cyclic peptide on a proteinogenic structure, that is, on a protein without imposing a limitation on the cyclic peptide and the protein.

A cyclic peptide has a chemically crosslinked structure for forming an intramolecular cyclic structure so that it is a peptide whose cyclic structure is formed by the chemically crosslinked structure. The present invention is characterized in the technical concept that the loop structure of a protein is used instead of the chemically crosslinked structure, in other words, a protein regarded as one molecule is used instead of the chemically crosslinked structure so far used for cyclizing a peptide.

However, the primary structure itself of the amino acid sequence of a modified protein obtained by fusing a cyclic peptide to a protein having a loop structure is not cyclized. As a whole, the structure derived from the cyclic peptide in the modified protein is apparently different from the original cyclic structure of the cyclic peptide but partially, the three-dimensional structure of the peptide is maintained and at the same time, the activity of the cyclic peptide can be maintained.

As an advantage of the present invention, it can be confirmed that an amino acid sequence derived from a cyclic peptide is presented on a protein having a loop structure while maintaining the structure of the cyclic peptide indirectly by whether, in a modified protein obtained by fusing the cyclic peptide onto the protein having a loop structure, the cyclic peptide maintains its original activity. In other words, in the present invention, it can be presumed that when the modified protein exhibits the activity which the cyclic peptide has, the cyclic peptide is presented on the protein having a loop structure while retaining its structure. In the present invention, whether or not the cyclic peptide maintains its structure may also be confirmed by acquiring the structure information of the modified protein.

Although the protein having a loop structure is not particularly limited, the following are examples of it. This protein may hereinafter be called "scaffold protein".

Examples of the scaffold protein are not particularly limited, but include immunoglobulin, fibronectin, albumin, human growth hormone, alkaline phosphatase, retinol-binding protein, uteroglobin, fibrinogen, blood coagulation factor, transferrin, genome editing enzyme containing Cas9, G protein conjugated receptor, cytokine receptor, growth factor receptor, integrin, cadherin, transferrin receptor, immunoreceptor, viral envelope protein, and viral capsid protein.

The scaffold protein may be a partial fragment protein and examples of it include the partial fragment protein of the above-exemplified proteins.

The scaffold protein may be a partial fragment containing a loop structure as a structure of a portion in the full-length protein insofar as it has a loop structure.

The scaffold protein may be that obtained by fusing, to a protein having a loop structure or its partial fragment protein having a loop structure, another protein.

The scaffold protein exemplified above has, in the secondary structure thereof, a loop structure.

The loop structure is not particularly limited and it means a structure which is a polypeptide chain region in which α helices and/or β sheets are linked and has a folded structure.

In the present invention, a loop structure present in each of the above-exemplified scaffold proteins can be used.

In the present invention, the cyclic peptide is inserted into the loop structure of the scaffold protein to fuse the cyclic peptide and the scaffold protein. This fusion is achieved preferably by covalent bonding.

The loop structure to be covalently bonded with the cyclic peptide is not particularly limited and it may be a loop structure present in the above-exemplified scaffold protein.

No particular limitation is imposed on the site of the loop structure into which the cyclic peptide is inserted and the cyclic peptide may be inserted into any position in the loop structure with versatility.

The term "any site in the loop structure" means that two amino acid residues for inserting the cyclic peptide are sites selected from the loop structure.

The two amino acid residues in the loop structure are, in the loop structure, present with a Cα atomic distance of from 4 to 7 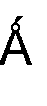 and are each at least partially exposed from the surface of the protein.

Since the Cα atomic distance of the two amino acid residues is from 4 to 7 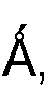 , the cyclic peptide fused to the scaffold protein can easily maintain its original structure of the peptide also in the modified peptide.

The Cα atomic distance of the two amino acid residues can also be confirmed from the structure data of the scaffold protein whose steric structure is known. Even if the steric structure of the scaffold protein is not known, the distance between the two amino acid residues can be estimated based on a model structure constructed from the structure information of a protein homologous to the scaffold protein (homolog).

The two amino acid residues selected for fusing to the cyclic peptide in the loop structure may be amino acid residues adjacent to each other in the loop structure but they are preferably amino acid residues not adjacent to each other. The term "two amino acids not adjacent to each other" means that two amino acid residues are discontinuous amino acid residues in the amino acid primary sequence in the loop structure of the protein.

For example, in the case where in a crystal structure of the protein, two amino acid residues arranged to have a Cα atomic distance of from 4 to 7 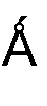 are selected, the two amino acid residues of the scaffold protein constituting the loop structure may have from 1 to 15 amino acid residues therebetween. The term "have from 1 to 15 amino acid residues therebetween" means that the two amino acid residues are present with from 1 to 15 amino acid residues therebetween in the amino acid primary sequence in the loop structure of the protein.

These 1 to 15 amino acid residues between the two amino acid residues are preferably amino acid residues forming a loop structure.

In the method of presenting an amino acid sequence derived from a cyclic peptide on a protein having a loop structure according to the present invention, the amino acid sequence derived from a cyclic peptide is presented on a protein having a loop structure and used as a scaffold. Preferably, the structure of the protein and the structure of the cyclic peptide can be maintained by replacing the loop structure with the cyclic peptide.

By selecting, from the loop structure, two amino acid residues to be bound to the cyclic peptide so as to adjust a Cα atomic distance of the two amino acid residues to from 4 to 7 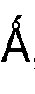 , it is possible to maintain the structure which the protein essentially has and to retain the activity of the cyclic peptide.

In the present invention, the term "retain the activity of the cyclic peptide" means as follows. A cyclic peptide to be fused to a protein having a loop structure is preferably known as a compound having some physiological activity and even after the cyclic peptide having physiological activity is fused to the protein, the cyclic peptide retains its physiological activity.

In this case, the cyclic peptide may have, as the degree of physiological activity, an activity value or inhibition value different between before and after fusion to the protein.

In the present invention, the term "replacing the chemically crosslinked structure of the cyclic peptide with two amino acid residues from the loop structure" means as follows.

In replacing with two amino acid residues constituting the loop structure, a structure other than the chemically crosslinked structure of the cyclic peptide may be bonded to two amino acid residues constituting the loop structure or a partial amino acid sequence obtained by removing the chemically crosslinked structure of the cyclic peptide and further removing one or more amino acids constituting the cyclic structure of the cyclic peptide may be bonded to the two amino acid residues.

Bonding of a partial amino acid residue may be bonding of a partial structure including a structure for exhibiting activity of the cyclic peptide, more specifically, physiological activity thereof.

The amino acids constituting the cyclic peptide may be, as shown below, proteinogenic amino acids and/or non-proteinogenic amino acids. It is preferred that the cyclic peptide partially contains a non-proteinogenic amino acid. When the cyclic peptide contains a non-proteinogenic amino acid, the non-proteinogenic amino acid derived from the cyclic peptide is preferably substituted by a proteinogenic amino acid in the modified protein to which the cyclic peptide has been fused.

When fused to the scaffold protein, the proteinogenic amino acid constituting the cyclic peptide is preferably fused as an amino acid as is, but it may be fused after substitution by another proteinogenic amino acid.

The amino acid sequence of the cyclic peptide to be fused may be the same as the amino acid sequence of the original cyclic peptide or may be a sequence obtained by substitution, deletion, or insertion of one or more amino acids.

In the present invention, the term "one or more amino acids" may mean 1, 2, 3, 4, 5, 6, 7, 8, 9 or 10 amino acids or from 1 to 10, from 1 to 9, from 1 to 8, from 1 to 7, from 1 to 6, from 1 to 5, from 1 to 4, from 1 to 3, from 1 to 2, or one amino acid.

In the present invention, in "replacing the chemically crosslinked structure of the cyclic peptide with two amino acid residues from the loop structure", the chemically crosslinked structure of the cyclic peptide may be replaced with two amino acid residues constituting the loop structure via a linker sequence.

When the chemically crosslinked structure is replaced through a linker sequence, the amino acid sequence derived from the loop structure, the linker sequence, and the amino acid sequence derived from the cyclic peptide are fused in order of mention to the N-terminal side of the modified protein to which the cyclic peptide has been fused, while the amino acid sequence derived from the cyclic peptide, the linker sequence, and the amino acid sequence derived from the loop structure are fused in order of mention to the C-terminal side of the modified protein.

The modified protein may have the linker sequence on both the N-terminal side and the C-terminal side, but it may have the linker sequence on either one of the N-terminal side and the C-terminal side.

The linker sequence is not particularly limited and examples include sequences composed of one or more Ser(s), Gly(s), and/or Cys(s), more specifically, sequences composed of from 1 to 5, from 1 to 4, from 1 to 3, from 1 to 2, or one Ser, Gly, and/or Cys. The linker sequence is preferably a sequence composed of Ser and/or Gly.

In the present invention, in "replacing the chemically crosslinked structure of the cyclic peptide with two amino acid residues from the loop structure", the chemically crosslinked structure of the cyclic peptide may be replaced with the two amino acid residues constituting the loop structure while including an amino acid sequence constituting the loop structure other than two amino acids constituting the loop structure.

When replacement is achieved while including an amino acid sequence constituting the loop structure other than the two amino acids constituting the loop structure, the N-terminal side one of the two amino acids constituting the loop structure, the amino acid sequence derived from the loop structure, a linker sequence if desired, and the amino acid sequence derived from the cyclic peptide are fused in order of mention to the N-terminal side of the modified protein to which the cyclic peptide has been fused, while the amino acid sequence derived from the cyclic peptide, a linker sequence if desired, the amino acid sequence derived from the loop structure, and the C-terminal side one of the two amino acids constituting the loop structure are fused in order of mention to the C-terminal side of the modified protein.

The modified protein may have, on both the N-terminal side and C-terminal side thereof, an amino acid sequence constituting the loop structure other than the two amino acids constituting the loop structure or it may have, on either the N-terminal side or the C-terminal side thereof, an amino acid sequence constituting the loop structure other than the two amino acids constituting the loop structure.

The term "amino acid sequence constituting the loop structure other than the two amino acids from the loop structure" is preferably a plurality of amino acid sequences derived from the loop structure sandwiched between the two amino acids constituting the loop structure and they are amino acid sequences adjacent to and bound to the two amino acids constituting the loop structure, respectively.

The following is a more specific description.

In the present invention, when of the two amino acid residues present in the scaffold protein to be replaced for the chemically crosslinked structure of the cyclic peptide, the amino acid residue constituting the loop structure and situated on the N-terminal side is represented by B_{N} and the amino acid residue constituting the loop structure and situated on the C-terminal side is represented by B_{C}, the loop structure of the cyclic peptide includes a structure represented by the following formula: -B_{N}-(Xaa1)ₘ-B_{C}- (whenever the cyclic peptide has the loop structure, Xaa1(s) each independently represent an arbitrary amino acid residue and m stands for an arbitrary integer of 0 or more, preferably an integer from 1 to 15).

In in "replacing the chemically crosslinked structure of the cyclic peptide with two amino acid residues from the loop structure", the amino acid derived from the cyclic peptide may be bound to B_{N}, may be bound to B_{N} via a linker sequence, or may be bound to the x1-th Xaa1, x1 meaning an arbitrary number counted from B_{N}, via a linker sequence if desired.

In in "replacing the chemically crosslinked structure of the cyclic peptide with two amino acid residues from the loop structure", the amino acid derived from the cyclic peptide may be bound to B_{C}, may be bound to B_{C} via a linker sequence, or may be bound to the x2-th Xaa1, x2 meaning an arbitrary number counted from B_{C}, via a linker sequence if desired.

When the amino acid derived from the cyclic peptide is bound to the x1-th Xaa1, x1 meaning an arbitrary number counted from B_{N}, via a linker sequence if desired or when the amino acid derived from the cyclic peptide is bound to the x2-th Xaa1, x2 meaning an arbitrary number counted from B_{C}, via a linker sequence if desired, the modified protein having the cyclic peptide fused therein has an amino acid sequence constituting the loop structure in addition to the two amino acids constituting the loop structure.

On the other hand, when of the two amino acid residues of the cyclic peptide constituting the chemically crosslinked structure for forming an intramolecular cyclic structure, the amino acid residue situated on the N-terminal side is represented by C_{N} and the amino acid residue on the C-terminal side is represented by C_{C}, the cyclic peptide has at least a primary sequence represented by the following formula: -Cₙ-(Xaa2)ₙ-C_{C}- (whenever the cyclic peptide can be formed, Xaa2(s) each independently represent an arbitrary amino acid residue and n stands for an arbitrary integer of 2 or more). The cyclic peptide may have a linear branched chain from the cyclic structure, in addition to the intramolecular cyclic structure. With respect to the cyclic peptide, C_{N} and C_{C} which are two amino acid residues constitute the chemically crosslinked structure for forming an intramolecular cyclic structure, thereby forming the cyclic peptide.

In "replacing the chemically crosslinked structure of the cyclic peptide with two amino acid residues from the loop structure", the two amino acids constituting the loop structure or an amino acid, other than the two amino acids constituting the loop structure, constituting the loop structure may be bound to C_{N} and/or C_{C}, via a linker sequence if desired.

When C_{N} and/or C_{C} is a non-proteinogenic amino acid, it may be substituted by a proteinogenic amino acid. When C_{N} and/or C_{C} is Cys, the C_{N} and/or C_{C} may be deleted. When C_{C} is Cys, C_{N} may be substituted by Cys and the cyclic peptide may be fused to it.

In "replacing the chemically crosslinked structure of the cyclic peptide with two amino acid residues from the loop structure", the amino acid derived from the cyclic peptide may be bound at C_{N}, may be bound at C_{N} via a linker sequence, or may be bound at y1-th Xaa2, y1 meaning an arbitrary number counted from C_{N}, via a linker sequence if desired. In particular, when C_{N} is a non-proteinogenic amino acid, after substitution of C_{N} by a proteinogenic amino acid, the amino acid derived from the cyclic peptide may be bound to it via a linker sequence if desired.

In "replacing the chemically crosslinked structure of the cyclic peptide with two amino acid residues from the loop structure", the amino acid derived from the cyclic peptide may be bound at C_{C}, may be bound at C_{C} via a linker sequence, or may be bound at y2-th Xaa2, y2 meaning an arbitrary number counted from C_{C}, via a linker if desired. In particular, the amino acid derived from the cyclic peptide may be bound, after deletion of C_{C}, at the first Xaa2 counted from C_{C}, that is, at Xaa2 to be bound to C_{C} via a linker sequence if desired.

When the amino acid derived from the cyclic peptide is bound to the y1-th Xaa2, y1 meaning an arbitrary number counted from C_{N}, via a linker if desired, or when it is bound to y2-th Xaa2, y2 meaning an arbitrary number counted from C_{C}, by a linker if desired, the modified protein having the cyclic peptide fused therein includes a partial sequence of the cyclic peptide.

The term "a cyclic peptide is fused to a protein having a loop structure" means that the whole of -(Xaa1)ₘ- of the loop structure -B_{N}-(Xaa1)ₘ-B_{C}- or a portion -(Xaa1)ₚ- thereof is replaced with the whole or a portion of C_{N}-(Xaa2)ₙ-C_{C} constituting the intramolecular cyclic structure of the cyclic peptide.

The structure after replacement becomes a structure represented by - B_{N}-(Xaa1)_{q}-[C_{N}-(Xaa2)ₙ-C_{C}]-(Xaa1)ᵣ-B_{C}-. This structure does not include a linker sequence (p is an integer not more than m, p+q+r is m, q and r are an integer of 0 or more, preferably an integer from 0 to 10). When it has a linker sequence, a bond of (Xaa1)_{q}-[Cₙ-(Xaa2)ₙ-C_{C}] and a bond of [C_{N}-(Xaa2)ₙ-C_{C}]-(Xaa1)ᵣ have therebetween the linker sequence.

It is to be noted that [C_{N}-(Xaa2)ₙ-C_{C}] means that all or some of amino acid sequences in the sequence are fused. Preferably, C_{N}-(Xaa2)ₙ is fused as a partial sequence. When C_{N} and/or C_{C} is a non-proteinogenic amino acid, they may be regarded as a proteinogenic amino acid in fusion.

As the amino acid residue represented by B_{N} and B_{C}, an amino acid residue in the loop structure may be used as is or alternatively, it may be substituted by another amino acid residue. As the amino acid residue represented by C_{N} and C_{C}, an amino acid residue in the cyclic peptide may be used as is, may be substituted by another amino acid residue or may be deleted. Preferably, C_{N} is substituted by a proteinogenic amino acid and C_{C} is deleted.

In the modified protein having the cyclic peptide fused therein, the number of amino acids between the N-terminal amino acid in the amino acid sequence derived from the cyclic peptide and B_{N} which is one of the two amino acid residues constituting the loop structure and fused on the N-terminal side and the number of amino acids between the C-terminal amino acid in the amino acid sequence derived from the cyclic peptide and B_{C} which is one of the two amino acid residues constituting the loop structure and fused on the C-terminal side may be the same or different.

The number of amino acids between the N-terminal amino acid in the amino acid sequence derived from the cyclic peptide and B_{N} which is one of the two amino acid residues constituting the loop structure and fused on the N-terminal side may be 0, 1, 2, 3, 4, 5, 6, 7, 8, 9, or 10 or may be from 0 to 10, from 0 to 9, from 0 to 8, from 0 to 7, from 0 to 6, from 0 to 5, from 0 to 4, from 0 to 3, from 0 to 2, from 0 to 1, or 0 amino acid.

The number of amino acids between the C-terminal amino acid in the amino acid sequence derived from the cyclic peptide and B_{C} which is one of the two amino acid residues constituting the loop structure and fused on the C-terminal side may be 0, 1, 2, 3, 4, 5, 6, 7, 8, 9, or 10 or may be from 0 to 10, from 0 to 9, from 0 to 8, from 0 to 7, from 0 to 6, from 0 to 5, from 0 to 4, from 0 to 3, from 0 to 2, from 0 to 1, or 0 amino acid.

The cyclic peptide may be a peptide produced by the typical mRNA display method, may be a peptide produce by the TRAP or RaPID method, or may be a peptide produced by the phage display method. The cyclic peptide may be a peptide produced by a modified method thereof.

The cyclic peptide contains, for example, a thioether bond or a disulfide bond as the chemically crosslinked structure for forming an intramolecular cyclic structure.

Typically in the cyclic peptide selected by the mRNA display method such as RaPID method or TRAP method or the phage display method, a structure other than the chemically crosslinked structure for forming an intramolecular cyclic structure such as thioether bond or disulfide bond tends to be an active spot having physiological activity.

By replacing the thioether bond or disulfide bond of the cyclic peptide with a bond with a protein having a loop structure, high specificity and affinity of the cyclic peptide ordinarily available by the mRNA display method such as RaPID method or TRAP method or the phage display method can be bestowed into an intended loop structure of an intended protein. Although not particularly limited, versatile fusion of a cyclic peptide can be achieved by fusing a cyclic peptide having a thioether bond or disulfide bond as an intramolecular cyclic structure.

The cyclic peptide is not particularly limited and it may be either a naturally occurring cyclic peptide or a non-naturally occurring cyclic peptide.

When a naturally occurring cyclic peptide is presented on a protein, any bond for binding amino acid residues to each other may be used because they can be regarded as a chemically crosslinked structure for forming an intramolecular cyclic structure. Bonds between amino acid residues in a region other than a region thought to be an active site in a naturally occurring cyclic peptide are however used preferably as a chemically crosslinked structure for fusing to a protein.

The cyclic peptide is a peptide having, in the molecule thereof, at least a cyclic structure composed of four or more amino acid residues. The cyclic structure of the cyclic peptide composed of four or more amino acid residues is a closed-ring structure formed in the molecule by bonding, directly or via a linker or the like, two amino acid residues of a linear peptide separated by two or more amino acids.

The term "two amino acid residues separated by two or more amino acids" means that the two amino acid residues have at least two amino acid residues therebetween. The two amino acid residues bond to each other while having two or more amino acids therebetween.

The closed-ring structure in the cyclic structure is not particularly limited but it is formed by the covalent bond between two amino acids.

Examples of the covalent bond between two amino acids include disulfide bond, peptide bond, alkyl bond, alkenyl bond, ester bond, thioester bond, ether bond, thioether bond, phosphonate ether bond, azo bond, C-S-C bond, C-N-C bond, C=N-C bond, amide bond, lactam bridge, carbamoyl bond, urea bond, thiourea bond, amine bond, and thioamide bond.

When two amino acids bond to each other at their main chain, a closed ring structure is formed by a peptide bond, but a covalent bond between two amino acids may be formed by bonding between the respective side chains of the two amino acids, bonding between the side chain and the main chain of them, or the like.

The cyclic structure is not limited to that formed by bonding between the N-terminal amino acid and the C-terminal amino acid of a linear peptide, but it may be formed by bonding between a terminal amino acid and a non-terminal amino acid or bonding between non-terminal amino acids. When one of the amino acids bonded for the formation of a cyclic structure is a terminal amino acid and the other one is a non-terminal amino acid, the resulting cyclic peptide has a cyclic structure having, as a linear branched chain from the cyclic structure, a linear peptide attached thereto like a tail.

The amino acid that forms a cyclic structure may be a proteinogenic amino acid, an artificial amino acid mutant, or a derivative thereof. Examples include proteinogenic L-amino acids and chemically synthesized compounds having properties known in the art as characteristics of an amino acid.

The proteinogenic amino acids are, when represented by three-letter code known in the art, Arg, His, Lys, Asp, Glu, Ser, Thr, Asn, Gln, Cys, Gly, Pro, Ala, Ile, Leu, Met, Phe, Trp, Tyr, and Val.

The term "non-proteinogenic amino acids" means naturally occurring or non-naturally occurring amino acids other than proteinogenic amino acids.

Examples of the non-naturally occurring amino acids include α,α-disubstituted amino acids (such as α-methylalanine), N-alkyl-α-amino acids, D-amino acids, β-amino acids, and α-hydroxy acids, each having a main chain structure different from that of naturally occurring amino acids; amino acids (such as norleucine and homohistidine) having a side-chain structure different from that of naturally occurring amino acids; amino acids (such as "homo" amino acids, homophenylalanine, and homohistidine) having extra methylene in the side chain thereof; and amino acids (such as cysteic acid) obtained by substituting a carboxylic acid functional group in the side chain by a sulfonic acid group. Specific examples of the non-naturally occurring amino acids include amino acids described in WO2015/030014.

The number of amino acids constituting the cyclic structure is not particularly limited insofar as it is 4 or more. It may be, for example, 5 or more, 8 or more, or 10 or more and may be 30 or less, 25 or less, 20 or less, or 15 or less.

The number of amino acids constituting the cyclic structure is preferably 4 or more to 30 or less. Within a range of 4 or more to 30 or less, the number of amino acids constituting the cyclic structure may be set at 5 or more, 8 or more, or 10 or more and set at 30 or less, 25 or less, 20 or less, or 15 or less.

The number of amino acids constituting the cyclic structure may be set at 8 or more to 20 or less, 10 or more to 20 or less, or 10 or more to 15 or less.

The cyclic peptide to be used in the present invention is a cyclic peptide that can be produced using a known peptide synthesis technology.

Examples of the method of producing the cyclic peptide include a chemical synthesis method such as liquid-phase method, solid-phase method, or hybrid method using a liquid-phase method and a solid-phase method in combination; a gene recombination method, and a translation synthesis method in a cell-free translation system.

The cyclic peptide is preferably a peptide that can be produced preferably by the mRNA display method such as RaPID method or TRAP method or the phage display method.

The RaPID method can produce, for example, a cyclic peptide in which an amino acid having Functional group 1 and an amino acid having Functional group 2 corresponding thereto, each shown below in Table 1 have been cyclized.

Either Functional group 1 or 2 may be placed on the N-terminal side; they may be placed at the N-terminal and C-terminal, respectively; one of them may be a terminal amino acid and the other one may be a non-terminal amino acid; or both may be a non-terminal amino acid.

A bond formed between Functional group 1 and Functional group 2 can be regarded as a chemically crosslinked structure for forming an intramolecular cyclic structure in a cyclic peptide.

**Table 1:**

| | Functional group 1 | Functional group 2 |
|---|---|---|
| (A) | 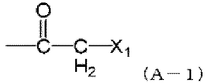 | HS- (A-2) |
| (B) | -C≡C-H (B-1) | N₃- (B-2) |
| (C) | -Ar-C**H**₂NH₂ (C-1) | 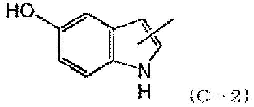 |
| (D) | -C≡C-C**H**₂-X₁ (D-1) | HS- (D-2) |
| (E) | -**Ar**-**CH**₂-X₁ (E-1) | HS- (E-2) |

In the above formulas, X₁ represents a leaving group, for example, a halogen atom such as Cl, Br, or I and Ar represents a substituted or unsubstituted aromatic ring.

In the phage display method, a cyclic peptide in which Cys and Cys bond to each other to form a cycle can be obtained so that a cyclic peptide having a disulfide bond between -SH as Functional group 1 and HS- as Functional group 2 can be obtained.

As the amino acid having Functional group (A-1), for example, a chloroacetylated amino acid can be used. Examples of the chloroacetylated amino acid include N-chloroacetyl-L-alanine, N-chloroacetyl-L-phenylalanine, N-chloroacetyl-L-tyrosine, N-chloroacetyl-L-tryptophan, N-3-(2-chloroacetamido)benzoyl-L-phenylalanine, N-3-(2-chloroacetamido)benzoyl-L-tyrosine, N-3-(2-chloroacetamido)benzoyl-L-tryptophan, β-N-chloroacetyl-L-diaminopropanoic acid, γ-N-chloroacetyl-L-diaminobutyric acid, σ-N-chloroacetyl-L-ornithine, and ε-N-chloroacetyl-L-lysine, and D-amino acid derivatives corresponding thereto.

As the amino acid having a functional group (A-1), N-chloroacetyl-L-tryptophan and N-chloroacetyl-L-tyrosine are preferably used, with D-form of it being more preferred.

In the present specification, an amino acid is sometimes described while showing clearly that it is in L-form but it may be either L-form or D-form. It may also be a mixture of L-form and D-form at any ratio. Even when an amino acid is described without clearly showing that it is in L-form or D-form, it may be either L-form or D-form, or a mixture of L-form and D-form at any ratio.

Examples of the amino acid having Functional group (A-2) include cysteine, homocysteine, mercaptonorvaline, mercaptonorleucine, 2-amino-7-mercaptoheptanoic acid, and 2-amino-8- mercaptooctanoic acid.

As the amino acid having Functional group (A-1), cysteine is preferably used.

The cyclization method using the amino acid having Functional group (A-1) and the amino acid having Functional group (A-2) may be carried out according to the method described, for example, in Kawakami, T. et al., Nature Chemical Biology 5, 888-890 (2009);Yamagishi, Y. et al., ChemBioChem 10, 1469-1472 (2009); Sako, Y. et al., Journal of American Chemical Society 130, 7932-7934 (2008); Goto, Y. et al., ACS Chemical Biology 3, 120-129 (2008); and Kawakami T. et al, Chemistry & Biology 15, 32-42 (2008), and WO2008/117833.

As the amino acid having Functional group (B-1), for example, propargylglycine, homopropargylglycine, 2-amino-6-heptynoic acid, 2-amino-7-octynoic acid, and 2-amino-8-nonynoic acid can be used.

Alternatively, a 4-pentynoylated or 5-hexynoylated amino acid may be used.

Examples of the 4-pentynoylated amino acid include N-(4-pentenoyl)-L-alanine, N-(4-pentenoyl)-L-phenylalanine, N-(4-pentenoyl)-L-tyrosine, N-(4-pentenoyl)-L-tryptophan, N-3-(4-pentynoylamido)benzoyl-L-phenylalanine, N-3-(4-pentynoylamido)benzoyl-L-tyrosine, N-3-(4-pentynoylamido)benzoyl-L-tryptophan, β-N-(4-pentenoyl)-L-diaminopropanoic acid, γ-N-(4-pentenoyl)-L-diaminobutyric acid, σ-N-(4-pentenoyl)-L-ornithine, and ε-N-(4-pentenoyl)-L-lysine, and D-amino acid derivatives corresponding thereto.

Examples of the 5-hexynoylated amino acid include amino acids obtained by substituting the 4-pentynoyl group of the compounds exemplified as the 4-pentynoylated amino acid by a 5-hexynoyl group.

As the amino acid having Functional group (B-2), for example, azidoalanine, 2-amino-4-azidobutanoic acid, azidoptonorvaline, azidonorleucine, 2-amino-7-azidoheptanoic acid, and 2-amino-8-azidooctanoic acid can be used.

Alternatively, azidoacetylated or 3-azidopentanoylated amino acid can also be used.

Examples of the azidoacetylated amino acid include N-azidoacetyl-L-alanine, N-azidoacetyl-L-phenylalanine, N-azidoacetyl-L-tyrosine, N-azidoacetyl-L-tryptophan, N-3-(4-pentynoylamido)benzoyl-L-phenylalanine, N-3-(4-pentynoylamido)benzoyl-L-tyrosine, N-3-(4-pentynoylamido)benzoyl-L-tryptophan, β-N-azidoacetyl-L-diaminopropanoic acid, γ-N-azidoacetyl-L-diaminobutyric acid, σ-N-azidoacetyl-L-ornithine, and ε-N-azidoacetyl-L-lysine, and D-amino acid derivatives corresponding thereto.

Examples of the 3-azidopentanoylated amino acid include amino acids obtained by substituting the azidoacetyl group of the compounds exemplified as the azidoacetylated amino acid by a 3-azidopentanoyl group.

Examples of the cyclization method using the amino acid having Functional group (B-1) and the amino acid having Functional group (B-2) include the method described in Sako, Y. et al., Journal of American Chemical Society 130, 7932-7934 (2008) and that described in WO2008/117833.

Examples of the amino acid having Functional group (C-1) include N-(4-aminomethyl-benzoyl)-phenylalanine (AMBF) and 3-aminomethyltyrosine.

Examples of the amino acid having Functional group (C-2) include 5-hydroxytryptophan (WOH).

Examples of the cyclization method using the amino acid having Functional group (C-1) and the amino acid having Functional group (C-2) include the method described in Yamagishi, Y. et al., ChemBioChem 10, 1469-1472 (2009) and that described in WO2008/117833.

Examples of the amino acid having Functional group (D-1) include 2-amino-6-chloro-hexynoic acid, 2-amino-7-chloro-heptynoic acid, and 2-amino-8-chloro-octynoic acid.

Examples of the amino acid having Functional group (D-2) include cysteine, homocysteine, mercaptonorvaline, mercaptonorleucine, 2-amino-7-mercaptoheptanoic acid, and 2-amino-8-mercaptooctanoic acid.

Examples of the cyclization method using the amino acid having Functional group (D-1) and the amino acid having Functional group (D-2) include the method described in WO2012/074129.

Examples of Amino acid (E-1) include N-3-chloromethylbenzoyl-L-phenylalanine, N-3-chloromethylbenzoyl-L-tyrosine, and N-3-chloromethylbenzoyl-L-tryptophan and D-amino acid derivatives corresponding thereto.

Examples of Amino acid (E-2) include cysteine, homocysteine, mercaptonorvaline, mercaptonorleucine, 2-amino-7-mercaptoheptanoic acid, and 2-amino-8- mercaptooctanoic acid.

The cyclization method of the amino acid having Functional group (E-1) and the amino acid having Functional group (E-2) can be carried out referring to, for example, the cyclization method using (A-1) and (A-2) or the cyclization method using (D-1) and (D-2).

The ring-forming amino acid is preferably combination of the amino acid having Functional group (A-1) with the amino acid having Functional group (A-2), more preferably combination of N-acetyltryptophan obtained by substituting H by a leaving group with cysteine, still more preferably combination of an N-haloacetyl-D-tyrosine or an N-haloroacetyl-D-tryptophan, preferably, N-chloroacetyl-D-tyrosine or N-chloroacetyl-D-tryptophan with cysteine.

In the present invention, it is preferred to use a cyclic peptide produced by the mRNA display method such as RaPID or TRAP or the phage display method and having physiological activity on an intended binding molecule.

It is preferred to fuse a bond, which is formed between Functional group 1 and Functional group 2 in a peptide produced preferably by the mRNA display method such as RaPID method or TRAP method or the phage display method, as a chemically crosslinked structure for forming an intramolecular cyclic structure, to a protein having a loop structure.

The cyclic peptide to be used in the present invention will hereinafter be described with a cyclic peptide produced by the RaPID method as an example.

In the present invention, the cyclic peptide produced by the RaPID method is not particularly limited but a cyclic peptide represented by the following formula can be given as an example.

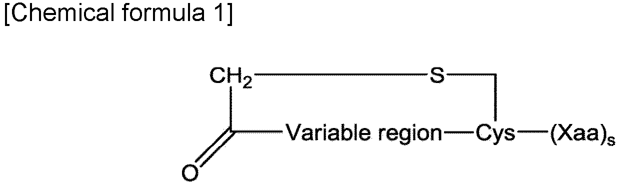

The cyclic peptide represented by the above formula is an example and it has a structure having, as Functional group 1 and Functional group 2, those listed in (A) in Table 1.

In the formula, S represents a sulfur atom derived from the thiol group of Cys, Xaa represents an arbitrary amino acid, and s stands for an arbitrary integer of 0 or more. The term "variable region" means an amino acid sequence, other than Cys, constituting a cyclic amino acid. The N-terminal amino acid of the variable region is preferably an amino acid having Functional group (A-1).

The amino acid sequence of the variable region is a partial amino acid sequence in the cyclic peptide produced by the RaPID method and it may be any amino acid sequence insofar as it constitutes the cyclic peptide.

When the peptide represented by the above formula is used as the cyclic peptide, the N-terminal amino acid residue of the variable region is C_{N} and Cys is C_{C}.

When C_{N} is, for example, an N-haloroacetyl-D-tryptophan which is a non-proteinogenic amino acid, it is preferably substituted with L-tryptophan which is a proteinogenic amino acid, while when it is, for example, N-chloroacetyl-D-tyrosine which is a non-proteinogenic amino acid, it is preferably substituted with L-tyrosine which is a proteinogenic amino acid. Then, the cyclic peptide is fused. Alternatively, C_{N} may be substituted with Cys which is a proteinogenic amino acid. When C_{C} is, for example, Cys, the cyclic peptide is preferably fused after deletion of C_{C}. When C_{C} is Cys and Cys which is C_{C} is also fused, fusion may be performed after substitution of C_{N} by Cys. When the cyclic peptide represented by the above formula is fused, D-amino acid is substituted by L-amino acid if desired but it is preferred that the amino acid sequence of Variable region is, as a partial amino acid sequence of the amino acid sequence constituting the cyclic structure of the cyclic peptide, fused to a modified protein having the cyclic peptide fused therein. In the present invention, it is understood that a portion of the amino acid sequence of Variable region - for example, the amino acid sequence of Variable region even when C_{N}, a non-proteinogenic amino acid, is substituted by a proteinogenic amino acid - is fused to the modified protein having the cyclic peptide fused therein. Variable region and Cys as C_{C} may also be fused together.

In order to fuse the cyclic peptide preferably produced by the mRNA display method such as RaPID method or TRAP method or the phage display method to the protein having a loop structure, it is preferred to carry out modification as described below.
(1) Amino acid residues involved in the chemically crosslinked structure for forming an intramolecular cyclic structure in the mRNA display method such as RaPID or TRAP or the phage display method are substituted or removed and the amino acid residue derived from the cyclic peptide may be fused to the amino acid residue derived from the loop structure.

More specifically, in the cyclic peptide produced by RaPID, the amino acid residue having Functional group 1 is substituted by a proteinogenic amino acid and the amino acid residue having Functional group 2 is deleted, followed by fusion to the loop structure. As the amino acid residue having Functional group 1, a non-proteinogenic amino acid such as D-amino is sometimes used.

In the cyclic peptide produced by the phage display method, a Cys residue constituting an S-S bond as the chemically crosslinked structure may be removed, followed by fusion to the loop structure.

More specifically, in the RaPID method, when the structure (A-1) is used for Functional group 1, it is the common practice to use CIAc-D-Trp or ClAc-D-Tyr as an amino acid having the structure (A-1), but in a modified protein, the amino acid residue is preferably substituted by L-Trp or L-Tyr. Alternatively, CIAc-D-Trp or CIAc-D-Tyr may be deleted.

As the amino acid having the structure (A-2), Cys is often used, but the Cys residue may be deleted from the modified protein.

(2) In (1), an amino acid sequence not having a cyclic peptide but composed of an amino acid residue such as Ser, Gly and Cys is used as a linker sequence between the cyclic peptide and the loop structure and the amino acid residue derived from the cyclic peptide may be fused to the amino acid residue derived from the loop structure while inserting the linker sequence therebetween.

(3) In a modified protein obtained by converting an amino acid residue involved in the chemically crosslinked structure for forming an intramolecular cyclic structure into L-Cys and fusing the cyclic peptide to the loop structure, a crosslinked structure may be formed by disulfide bonding and at the same time, an amino acid sequence composed of an amino acid residue such as Ser and Gly is used as a linker sequence between the cyclic peptide and the loop structure and is inserted between the amino acid residue derived from the cyclic peptide and the amino acid residue derived from the loop structure to fuse them.

The number of the amino acid residues constituting the linker sequence may be one or more and the number of the amino acid residues is not particularly limited.

In the present invention, the method of presenting an amino acid sequence derived from a cyclic peptide on a protein having a loop structure includes fusing the cyclic peptide to the protein having a loop structure. The cyclic peptide can be fused to the protein having a loop structure by typical gene engineering technology.

More specifically, the present invention provides, as a method of fusing a partial amino acid sequence derived from a cyclic peptide to a protein, a method of fusing a partial amino acid sequence derived from a cyclic peptide to a protein having a loop structure, thereby producing a modified protein having the cyclic peptide presented on the protein.

The method of fusing a partial amino acid sequence derived from a cyclic peptide to a protein having a loop structure, thereby producing a modified protein having the cyclic peptide presented on the protein, includes:
using, as the cyclic peptide, a cyclic peptide having a chemically crosslinked structure for forming an intramolecular cyclic structure,
selecting, from the amino acid sequence of the cyclic peptide, a partial amino acid sequence to be presented on the protein and selecting a base sequence corresponding to the partial amino acid sequence,
selecting base sequences corresponding to two amino acid sequences constituting the loop structure of the protein having a loop structure, respectively, removing a base present between the selected base sequences if necessary, and preparing a nucleic acid having, inserted and incorporated therein, the base sequence corresponding to the partial amino acid sequence of the cyclic peptide, and translating the nucleic acid.

As the cyclic peptide, a cyclic peptide selected by the mRNA display method such as RaPID or TRAP or the phage display method is preferred, with that selected by the mRNA display method being more preferred. The base sequence of the partial amino acid sequence of the cyclic peptide to be inserted can be easily understood by the mRNA display method or the like.

A method of selecting base sequences corresponding to two amino acid residues constituting the loop structure of the protein having a loop structure, respectively, and removing a base present between the selected base sequences if necessary or a method of preparing a nucleic acid having, inserted and incorporated therein, the base sequence corresponding to the partial amino acid sequence of the cyclic peptide is not particularly limited and can be carried out using a conventionally known method.

A method of translating the nucleic acid thus prepared is well known in the technical field to which the present invention belongs so that translation of the nucleic acid may be performed using such a well-known method as needed.

In selecting base sequences corresponding to two amino acid residues constituting the loop structure of the protein having a loop structure, two amino acid residues which will become scaffolds for fusing the cyclic peptide thereto are selected from amino acid sequences constituting the loop structure in amino sequences of the protein having a loop structure (may be amino acid sequences of a partial segment protein or amino acid sequences of a fusion protein).

The two amino acid residues can be selected by using the structure data of a scaffold protein or by using a model structure constructed based on the structure information of a protein (homolog) analogous thereto even if the steric structure of the scaffold protein is unknown.

In selecting the two amino acid residues, when a scaffold protein having a plurality of loop structures is selected, it is preferred to select a loop structure to which the cyclic peptide is fused and in the present invention, two amino acid residues having a Cα atomic distance of from 4 to 7 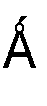 from the loop structure are selected.

In selecting two amino acid residues, it is preferred to select two amino acid residues separated from each other by from 1 to 15 amino acid residues and in the present invention, two amino acid residues having a Cα atomic distance of from 4 to 7 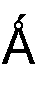 are selected, which are preferably separated from each other by from 1 to 15 amino acid residues.

In the two amino acid residues selected from the scaffold protein, the N-terminal side amino acid residue is represented by B_{N} and the C-terminal side amino acid residue is represented by B_{C}, each in the original scaffold protein.

The C-terminal side amino acid sequence to be bound to B_{N} may be used for binding to the cyclic peptide and the N-terminal side amino acid sequence to be bound to B_{C} may be used for binding to the cyclic peptide.

After the amino acid residues to be bound to the cyclic peptide are selected, addition of a linker sequence may be selected.

In selecting the cyclic peptide to be fused with the scaffold protein, a cyclic peptide known to have some physiological activity or a cyclic peptide confirmed to have physiological activity is preferably selected. The amino acid sequence of a cyclic peptide may be confirmed as needed by a conventional method.

The amino acid sequence of the cyclic peptide to be fused is selected based on the information of a primary amino acid sequence available by identifying a bond to be a chemically crosslinked structure of the cyclic peptide and cutting the bond.

A predetermined modified protein can be produced by determining a base sequence necessary for the translation of the protein based on the amino acid sequence thus selected and carrying out translation.

The modified protein obtained according to the method of the present invention by using a protein having a loop structure as a scaffold and presenting a cyclic peptide on the protein can be used as a reagent, a drug, or the like based on the activity of the cyclic peptide, because the cyclic peptide is presented while maintaining the structure and activity of the cyclic peptide.

The modified protein having the cyclic peptide fused therein exhibits both the activity of the protein itself and the activity of the cyclic peptide so that it can be used as a modified protein having two different activities or activities of the same kind.

In the present invention, one cyclic peptide may be fused to the one or more loop structures of a protein having one or more loop structures or two or more cyclic peptides may be fused to the respectively different loop structures of a protein having a plurality of loop structures.

When two or more cyclic peptides are fused, they may be cyclic peptides having the same amino acid sequence or two or more cyclic peptides may be cyclic peptides having respectively different amino acid sequences.

### Examples

The present invention will hereinafter be described specifically by Examples, but the present invention is not limited to or by the following Examples.

Human plexin B1, human Met receptor, human EGF receptor, and human TrkB receptor to be used as a binding molecule were subjected to RaPID system based on WO2011/049157 and Japanese Patent Application Laid-Open No. 2013-46637 and 1 to 3 cyclic peptides specifically binding to each of them were obtained. The structure of the cyclic peptides is shown in FIG. 1.

In FIG. 1, the N-terminal amino acid of the amino acid sequence of Variable region and Cys (shown in the formula) of the constant region respectively correspond to amino acid residues C_{N} and C_{C} to be used for linkage later. D-amino acids represented respectively by small letters w and y as the N-terminal amino acids of the cyclic peptides are introduced into a fusion protein after substitution into an L-amino acid or deletion.

Of the cyclic peptides listed in FIG. 1, P6 and P7 are cyclic peptides described in Cell Chem. Biol, 2016, 23, 1341-1350 and they each have binding activity to human Plexin B. A cyclic peptide mP6-9 is obtained by partially modifying the amino acid sequence of P6 and similar to P6, it binds to human Plexin B1. Cyclic peptides P6 and mP6-9 bind to human Plexin B1 to allosterically inhibit binding of Semaphorin 4D to human Plexin B1. As a result, they have activity of inhibiting morphologic change of cells caused by stimulation of Semaphorin 4D (Cell Chem. Biol, 2016, 23, 1341-1350).

Cyclic peptides aMD4, aMD5, and aML5 described in Nature Commun, 2015, 6, 6373 each have binding activity to a human Met receptor. It has been revealed that these peptides do not activate a human Met receptor alone, but after dimerized by crosslinking, they activate the human Met receptor and acquire agonist activity.

Cyclic peptides A6-2f and trkD5 respectively bind to a human EGF receptor and a human TrkB receptor with nanomole level affinity.

### [Example 1] Fusion between tenth Type III repeat domain of fibronectin and cyclic peptide

### 1. Design of cyclic peptide fusion protein

In order to replace with the chemically crosslinked structure of the above-described eight physiologically active cyclic peptides, the tenth Type III repeat domain (which will hereinafter be called "Fn10") of human fibronectin was selected as a protein having a loop structure and serving as a scaffold protein and two amino acid residues Val1490 (corresponding to B_{N}) and Ser1499 (corresponding to B_{C}) having a Cα atomic distance of 4.1Á were selected as linking residues from the loop portion sandwiched between the sixth and seventh β strands on the steric structure of the protein. The steric structure of the domain is shown in FIG. 2.

A fusion protein is designed by replacing a portion of eight amino acid residues sandwiched between two amino acid residues B_{N} and B_{C} selected in Fn10 with the amino acid sequence of the variable region of the cyclic peptide shown in FIG. 1 having, on both sides thereof, an arbitrary linker amino acid added as needed (the amino acid sequence of the variable region of the fused cyclic peptide is designed by substituting a non-proteinogenic amino acid of the variable region of the cyclic peptide represented by w or y by a proteinogenic amino acid W or Y, respectively. This will equally apply hereinafter. Only when P7 is fused, the N-terminal amino acid residue w in the variable region of P7 is substituted by Cys and this Cys is designed together with Cys corresponding to C_{C}). The amino acid sequence of the loop portion, after the cyclic peptide is fused, in the fusion protein thus designed is shown in FIG. 3.

### 2. Preparation of cyclic peptide fusion protein

DNA encoding the signal sequence of human prolactin, DNA encoding a region of amino acid residues Nos. 1418 to 1509 of human fibronectin, and DNA encoding the Fc region of human IgG1 were linked to form a construct of an Fn10-Fc fusion protein. The resulting construct was then incorporated in an expression vector pcDNA3.1 (product of Thermo Fisher Scientific).

By using the Fn10-Fc expression vector, an expression vector was prepared by replacing the eight amino acid residues of the loop region sandwiched between Val1490 and Ser1499 in the fibronectin region with each of the insertion sequences shown in FIG. 3. The insertion sequence portion was amplified using by the extension PCR method to obtain respective constructs of various intended cyclic peptide fusion proteins. The cyclic peptide fusion proteins thus obtained were called Fn10(name of cyclic peptide)-Fc, for example, Fn10(P6)-Fc.

Expi293F cells (product of Thermo Fisher Scientific) were seeded to give 3x10⁶ cells/mL on 3 mL of Expi293 Expression Medium (product of Thermo Fisher Scientific). Then, by the conventional method, gene introduction of 3 µm g of the Fn10-Fc (or a mutant thereof) expression vector into the Expi293F cells was performed using ExpiFectamine 293 Reagent (product of Thermo Fisher Scientific). After gene introduction, the cells were cultured with shaking for 18 hours at 125 rpm under the conditions of 37°C and 8% CO₂. Then, 15 µL of ExpiFectamime 293 Transfection Enhancer 1 and 150 µL of ExpiFectamime 293 Transfection Enhancer 2 (each, product of Thermo Fisher Scientific) were added and the resulting mixture was cultured with shaking for 3 days at 125 rpm under the conditions of 37°C and 8%CO₂. The resulting supernatant was then collected.

After 30 µL of Protein A-Sepharose (product of Thermo Fisher Scientific) was added to 0.3 mL of the supernatant thus collected, the resulting mixture was mixed by rotation for 2 hours. Sepharose was precipitated by centrifugal separation and the supernatant was removed. The Sepharose was then washed three times with 1 mL of Tris-buffered saline (TBS, 20 mM Tris-HCl, 150 mM NaCl, pH7.5). Then, 20 µL of an SDS sample buffer was added and the resulting mixture was heated at 95°C for 2 minutes to elute the sample. The sample thus eluted (5 µL) was subjected to electrophoresis under reducing conditions and then dyed with Coomassie Brilliant Blue.

The results of the electrophoresis are shown in FIG. 4. In FIG. 4, the sample number is shown by a number in circle. The band of Fn10-Fc (Sample No. 1) is observed at an expected molecular weight (37.5 kDa) and all the cyclic peptide fusion proteins (Samples Nos. 2 to 9) show a molecular weight slightly higher than that, which corresponds to that they have a total amino acid length longer by 12 to 20 residues. It has been confirmed that the cyclic peptide fusion proteins are expressed·secreted from the Expi293F cells in an amount comparable to that of Fn10-Fc having no peptide inserted therein.

### 3. Binding of cyclic peptide fusion protein to binding molecule

A pull-down type binding experiment was performed to study whether or not Fn10-Fc having various cyclic peptides fused therein retained its binding ability, which the cyclic peptides had before fusion, to binding molecules. In accordance with the method described in Protein Exp. Purification, 2014, 95, 240-247, respective constructs encoding fusion proteins having a PA tag (product of Wako Pure Chemical Industries) added to the C terminus of an extracellular region of four binding molecules (each, single-pass membrane receptor) were created and the resulting constructs were each incorporated in an expression vector pcDNA3.1 (product of Thermo Fisher Scientific). After transient expression in Expi293F cells by the above-described method while using the vector, respective culture supernatants containing Plexin B1-PA, Met-PA, EGFR-PA, and TrkB-PA, which were soluble receptor fragments, were prepared.

To 0.5 mL of the culture supernatant containing the PA-tag added receptor extracellular region, 30 µL of Sepharose (product of Wako Pure Chemical Industries) on which NZ-1, an anti-PA tag antibody, was immobilized and the resulting mixture was mixed by rotation for 2 hours to capture the binding molecule on Sepharose. After precipitation of Sepharose by centrifugation and removal of it, 0.5 mL of the separately-prepared culture supernatants containing various cyclic peptide fusion proteins (Fn10-Fc mutants), respectively was added and the resulting mixtures were mixed by rotation for 2 hours to cause a reaction. Centrifugation was performed again to precipitate Sepharose. The Sepharose was washed three times with 1 mL of TBS: By adding 20 µL of a SDS sample buffer and heating at 95°C for 2 minutes, a sample was eluted. A 5 µL portion of the sample thus eluted was subjected to electrophoresis under reducing conditions, followed by dyeing with Coomassie Brilliant Blue.

The results of the electrophoresis are shown in FIG. 5. Pulldown results have revealed that specific binding occurs between respective fusion proteins (Samples Nos. 2 to 4) having therein the amino acid sequences of Plexin binders P6, mP6-9, and P7 and Plexin B1-PA, between respective fusion proteins (Samples Nos. 5 to 7) having therein the amino acid sequences of Met binders aMD4, aMD5, and aML5 and Met-PA, between a fusion protein (Sample No. 8) having therein the amino acid sequence of an EGF receptor binder A6-2f and EGFR-PA, and between a fusion protein (Sample No. 9) having therein the amino acid sequence of a TrkB binder trkD5 and TrkB-PA.

The Fn10-Fc (Sample No. 1) used as a control did not show a binding property to any receptor protein. It has therefore been confirmed that by presenting on the loop structure of Fn10, any of the eight cyclic peptides can be converted into a fusion protein retaining the binding ability of the original cyclic peptide.

### [Example 2] Fusion between Fc region of antibody and cyclic peptide

### 1. Design of cyclic peptide fusion protein

In order to replace with the chemically crosslinked structure of a physiologically active cyclic peptide, a human IgG-derived Fc region (which will hereinafter be called "Fc") was selected as a protein having a loop structure and serving as a scaffold protein and two amino acid residues present within a Cα atomic distance of from 4 to 7 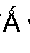 were searched from a loop portion sandwiched between β strands on its steric structure. As a result, eight sites were selected in total. They were three sites from "the upper portion" near the hinge region of Fc, three sites from "the bottom portion" of Fc, and two sites from "the side surface portion" of Fc. Sites used for fusion in "the upper portion" are called L1 site when two amino acid residues Ser 267 (corresponding to B_{N}) and Pro271 (corresponding to B_{C}) are used as linking residues, L2 site when two amino acid residues Tyr296 (corresponding to B_{N}) and Ser298 (corresponding to B_{C}) are used as linking residues, and L3 site when two amino acid residues Leu328 (corresponding to B_{N}) and Pro331 (corresponding to B_{C}) are used as linking residues. Sites used for fusion in "the bottom portion" are called L4 site when two amino acid residues Lys360 (corresponding to B_{N}) and Gln362 (corresponding to B_{C}) are used as linking residues, L5 site when two amino acid residues Ser383 (corresponding to B_{N}) and Gln386 (corresponding to B_{C}) are used as linking residues, and L6 site when two amino acid residues Gln419 (corresponding to B_{N}) and Asn421 (corresponding to B_{C}) are used as linking residues. Sites used for fusion in "the side surface portion" are called L7 site when two amino acid residues Gly341 (corresponding to B_{N}) and Pro343 (corresponding to B_{C}) are used as linking residues and L8 site when two amino acid residues Ser400 (corresponding to B_{N}) and Gly402 (corresponding to B_{C}) are used as linking residues. The steric structure of these portions is shown in FIG. 6.

A fusion protein was designed by replacing a portion of the region sandwiched between two amino acid residues B_{N} and B_{C} selected from the upper portion, bottom portion, or side surface portion of Fc with an amino acid sequence of the variable region of the cyclic peptide mP6-9, aMD4, or aMD5 having, on both sides of the amino acid sequence, an arbitrary linker amino acid added as needed. The amino acid sequence of the loop portion, after fusion of the cyclic peptide, of the fusion protein thus designed is shown in FIGS. 7 to 9.

### 2. Preparation of cyclic peptide fusion protein

A construct of an FC-fusion protein was created by linking DNA encoding the signal sequence of human prolactin, DNA encoding a Hisx8 tag, DNA encoding a Myc tag and DNA encoding the Fc region of human IgG1 and the resulting construct was incorporated in an expression vector pcDNA3.1 (product of Thermo Fisher Scientific).

Based on the Fc expression vector, an expression vector was prepared by replacing the amino acid sequence of the loop region of L1 to L8 sites with the insertion sequence shown in FIGS. 7 to 9. The insertion sequence portion was amplified using the extension PCR method to obtain respective constructs of various intended cyclic peptide fusion proteins. The cyclic peptide fusion proteins were each called Fc(name of cyclic peptide_name of site), for example, Fc(mP6-9_L1).

SDS-PAGE analysis was performed by carrying out transient expression of Fc and 20 cyclic peptide fusion proteins (three peptides and variation by 8 inserted sites) with Expi293F cells by the method described in Example 1 and causing precipitation of the culture supernatants thus obtained by using protein A Sepharose.

The results of electrophoresis are shown in FIG. 10. The band of Fc (Sample No. 1) was found at an expected molecular weight (30.6 kDa) and the band of all the cyclic peptide fusion proteins (Samples Nos. 2 to 21) appeared as a band showing mobility corresponding to a molecular weight of from 30 to 37 kDa.

3. Binding of cyclic peptide fusion protein to binding molecule Binding of the resulting cyclic peptide fusion proteins (Fc mutants) to NZ-1 Sepharose that had captured soluble receptor fragments Plexin B1-PA and Met-PA therein by the method described above in Example 1 was studied by the pulldown method.

The results of electrophoresis are shown in FIG. 11. The results of pulldown have revealed that all the eight fusion proteins (Samples Nos. 2 to 9) having therein the amino acid sequence of the Plexin binder mP6-9 bind to Plexin B1-PA and 11 (Samples Nos. 10 to 13 and 15 to 21), among 12 fusion proteins having therein the amino acid sequence of the Met binder, aMD4 or aMD5 specifically bind to Met-PA (specific binding of Fc (aMD5_L2, Sample No. 14) was also confirmed). In other words, it has been confirmed that as in the case of Fn10-Fc, the cyclic peptide can be converted into a fusion protein while retaining its binding activity to the binding molecule even by direct insertion of it into the loop structure of the Fc region of IgG.

### [Example 3] Fusion between IgG antibody and cyclic peptide

### 1. Design of cyclic peptide fusion protein

Human IgG full-length protein (which will hereinafter be called "IgG") was selected, as a protein having a loop structure and serving as a scaffold protein, to replace it with the chemically crosslinked structure of a physiologically active cyclic peptide. Since IgG included, as a portion of the structure thereof, Fc itself which was a scaffold protein used in Example 2, L1 to L8 sites shown in Example 2 were used as are as a site to be used for fusion. The steric structure of IgG and its Fc region (including a cyclic peptide fusion site) and two Fab regions which are antigen binding sites is shown in FIG. 12.

### 2. Preparation of cyclic peptide fusion protein

DNA encoding an H-chain full-length region of a human IgG1 monoclonal antibody to Neuropilin 1 was incorporated in an expression vector p3xFLAG-CMV-14 (product of Sigma Aldrich). DNA encoding the L_{K} chain full-length region of the above antibody was also incorporated in the p3xFLAG-CMV-14 vector similarly.

An H-chain expression vector was prepared by using the IgG1H chain expression vector, replacing the amino acid sequence of the loop region of L1 to L8 sites of the Fc region of the vector with the insertion sequence shown in FIGS. 7 to 9. The insertion sequence portion was amplified by the extension PCR method to obtain a construct of various intended cyclic peptide fusion H-chains. The cyclic peptide fusion proteins were each called IgG(name of peptide_name of site), for example, IgG(mp6-9_L1).

Each of the H-chain and its Plexin B1 binding cyclic peptide (mP6-9) fusion mutant expression vectors and an L_{K} chain expression vector corresponding thereto were mixed at 1:1. In a manner similar to that of Example 2, the resulting mixture was gene-introduced into Expi293F cells and transient expression was performed. Then, SDS-PAGE analysis was performed by precipitating the IgG protein thus expressed and secreted from the culture supernatant by using protein A Sepharose.

The results of electrophoresis are shown in FIG. 13A. The band of a fusion-free IgG (Sample No. 1) is observed at an expected molecular weight (H chain 47 kDa, L chain 25 kDa). In all the cyclic peptide fusion proteins (Samples Nos. 2 to 9), only the H-chain has a band showing mobility corresponding to a molecular weight slightly higher than 47kDa and the band of the L-chain appears at 25 kDa equal to that of IgG before fusion.

3. Binding of cyclic peptide fusion protein to binding molecule Binding of the resulting cyclic peptide fusion proteins (IgG mutants) to NZ-1 Sepharose that had captured a soluble receptor fragment Plexin B1-PA therein by the method described above in Example 2 was studied by the pulldown method.

The results of electrophoresis are shown in FIG. 13B. The results of pulldown have revealed that all of six fusion proteins (Samples Nos. 2 to 9) having, incorporated therein, the amino acid sequence of the Plexin binder mP6-9 show binding to Plexin B1-PA. It has been confirmed that as in single use of Fc, even if the cyclic peptide is directly inserted into the loop structure of the Fc region of IgG, the cyclic peptide can be converted into a fusion protein while retaining its binding activity to the binding molecule.

### [Example 4] Fusion between human serum albumin and cyclic peptide

### 1. Design of cyclic peptide fusion protein

In order to replace with the chemically crosslinked structure of a physiologically active cyclic peptide, human serum albumin (which will hereinafter be called "HSA") was selected as a protein having a loop structure and serving as a scaffold protein and two amino acid residues present within a Cα atomic distance of from 4 to 7 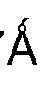 was searched from the loop portion sandwiched by α helices on the steric structure of the albumin. As a result, four sites having a high solvent exposure degree on the HSA molecule were selected. Sites to be used for fusion are called L1 site when two amino acid residues Asp56 (corresponding to B_{N}) and Ala59 (corresponding to B_{C}) are used as a linking residue, L2 site when two amino acid residues Cys169 (corresponding to B_{N}) and Lys174 (corresponding to B_{C}) are used as a linking residue, L3 site when two amino acid sequences Ala363 (corresponding to B_{N}) and Pro366 (corresponding to B_{C}), and L4 site when two amino acid residues Ala561 (corresponding to B_{N}) and Lys564 (corresponding to B_{C}) are used as a linking residue. The steric structure of the above portions is shown in FIG. 14.

A fusion protein was designed by replacing a portion of the region sandwiched between two amino acid residues B_{N} and B_{C} selected from HSA with the amino acid sequence, of the variable region of the cyclic peptide mP6-9 or aMD4, having on both sides thereof, an arbitrary linker amino acid as needed. The amino acid sequence of the loop portion, after fusion of the cyclic peptide, in the thus-designed fusion protein is shown in FIG. 15.

### 2. Preparation of cyclic peptide fusion protein

DNA encoding full-length human serum albumin, DNA encoding a Hisx8 tag, and DNA encoding a Myc tag were linked and a construct of an HSA fusion protein was created. It was incorporated in an expression vector pcDNA3,1 (product of Thermo Fisher Scientific).

An expression vector was prepared by using the HSA expression vector and replacing the amino acid sequence of the loop region of L1 to L4 sites with the insertion sequence shown in FIG. 12. The insertion sequence portion was amplified by the extension PCR method to obtain respective constructs of various intended cyclic peptide fusion proteins. The cyclic peptide fusion proteins were called HSA (name of cyclic peptide_name of site), for example, HSA(mP6-9_L1).

SDS-PAGE analysis of HSA and its eight cyclic peptide fusion proteins (two peptides × four variations of an inserted site) was performed by carrying out transient expression using Expi293F cells by the method described in Example 1 and causing precipitation with Ni-NTA agarose from the culture supernatant by making use of a His tag added to the C terminus.

The results of electrophoresis are shown in FIG. 16. The band of HSA (Sample No. 1) was found at an expected molecular weight (67 kDa) and the band of all the cyclic peptide fusion proteins (Samples Nos. 2 to 9) appeared as a band showing mobility corresponding to a molecular weight slightly higher than that of the above band.

3. Binding of cyclic peptide fusion protein to binding molecule Binding of the resulting cyclic peptide fusion proteins (HSA mutants) to a soluble receptor fragment of Plexin B1 or Met was confirmed by the method described above in Example 1. It is to be noted, however, that for the mP6-9 peptide fusion protein, not Plexin B1-PA but Plexin B1-Fc protein was used as a binding molecule and as pulldown beads, not NZ-1-Sepharose but protein A-Sepharose was used.

The results of electrophoresis are shown in FIG. 17. The pulldown results have revealed that all of the four fusion proteins (Samples Nos. 2 to 5) having, incorporated therein, the amino acid sequence of the Plexin binder mP6-9 binds to Plexin B1-Fc and all of the four fusion proteins (Samples Nos. 6 to 9) having, incorporated therein, the Met binder aMD4 specifically bind to Met-PA. It has been confirmed that in HSA (Sample No. 1) to which a cyclic peptide is not fused, only a non-specific band which can also be observed in a HSA-protein-free control culture supernatant (Sample No. 10) co-precipitates and the sample does not bind to the binding molecule. In other words, it has been confirmed that similar to the case of Fn10-Fc or Fc, the cyclic peptide can be converted into a fusion protein while retaining its binding activity to the binding molecule, even by directly inserting it into a loop sandwiched between α helices of HSA.

### [Example 5] Fusion between human growth hormone and cyclic peptide

### 1. Design of cyclic peptide fusion protein

In order to replace with the chemically crosslinked structure of a physiologically active cyclic peptide, human growth hormone (which will hereinafter be called "hGH") was selected as a protein having a loop structure and two amino acid residues present within a Cα atomic distance of from 4 to 7 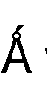 were searched from the loop portion sandwiched by α helices on the steric structure of the hormone. As a result, two sites corresponding to the top of the long axis were selected from on the molecular structure of hGH. Sites to be used for fusion are called L1 site when two amino acid residues Asp130 (corresponding to B_{N}) and Ser132 (corresponding to B_{C}) are used as a linking residue and L2 site when two amino acid residues Asn152 (corresponding to B_{N}) and Asp154 (corresponding to B_{C}) are used as a linking residue. The steric structure of the above portions is shown in FIG. 18.

A fusion protein was designed by replacing a portion of a region sandwiched between two amino acid residues B_{N} and B_{C} selected from hGH with the amino acid sequence of the variable region of the cyclic peptide mP6-9 having an arbitrary linker amino acid added as needed to both sides of the amino acid sequence. The amino acid sequence of the loop portion, after fusion of the cyclic peptide, of the designed fusion protein is shown in FIG. 19.

### 2. Preparation of cyclic peptide fusion protein

As a human growth hormone expression vector, pSGHV0 (described in Protein Expr. Purif., 2000, 20(3), 500-506) obtained by adding Hisx8 tag and TEV protease recognition sequence to the C terminus of the full-length hGH was used. An expression vector was prepared by using the above-described vector and replacing the amino acid sequence of the loop region of L1 and L2 sites with the insertion sequence shown in FIG. 19. The insertion sequence portion was amplified by the extension PCR method to obtain respective constructs of various intended cyclic peptide fusion proteins. The cyclic peptide fusion proteins were called hGH (name of cyclic peptide_name of site), for example, hGH(mP6-9_L1).

SDS-PAGE analysis of hGH and two cyclic peptide fusion proteins (one peptide × two variations of an inserted site) was performed by carrying out transient expression using Expi293F cells by the method described in Example 1 and causing precipitation with Ni-NTA agarose from the culture supernatant.

The results of electrophoresis are shown in FIG. 20A. The band of hGH (Sample No. 1) was found at an expected molecular weight (26 kDa) and the band of the two cyclic peptide fusion proteins (Samples Nos. 2 and 3) appeared as a band showing mobility corresponding to a molecular weight slightly higher than that of the above band.

### 3. Binding of cyclic peptide fusion protein to binding molecule

Binding of the resulting cyclic peptide fusion protein (hgH mutant) to NZ-1 Sepharose that had captured a soluble receptor fragment Plexin B1-PA therein by the method described above in Example 1 was studied by the pulldown method.

The results of electrophoresis are shown in FIG. 20B. The pulldown results have revealed that wild type hGH (Sample No. 1) does not bind to Plexin B1-PA, but two fusion proteins (Samples Nos. 2 and 3) having, incorporated therein, the sequence of the Plexin binder mP6-9 specifically binds to it. In other words, it has been confirmed that as in the case of Fn10-Fc, Fc, or HSA, the cyclic peptide can be converted into a fusion protein while retaining its binding activity to the binding molecule even by directly inserting it into the loop of a secretion hormone hGH.

### [Example 6] Fusion between human serum retinol-binding protein and cyclic peptide

### 1. Design of cyclic peptide fusion protein

In order to replace with the chemically crosslinked structure of a physiologically active cyclic peptide, human retinol binding protein (which will hereinafter be called "RBP") was selected as a lipid binding protein having a loop structure and serving as a scaffold protein and two amino acid residues present within a Cα atomic distance of from 4 to 7 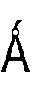 was searched from the loop portion sandwiched by β strands on the steric structure of the protein. As a result, two hairpin loop sites protruding from the β barrel structure of the RBP were selected. Sites to be used for fusion are called L1 site when two amino acid residues Leu 64 (corresponding to B_{N}) and Trp67 (corresponding to B_{C}) are used as a linking residue and L2 site when two amino acid residues Ala94 (corresponding to B_{N}) and Leu97 (corresponding to B_{C}) are used as a linking residue. The steric structure of the above sites is shown in FIG. 21.

A fusion protein was designed by replacing a portion of a region sandwiched between two amino acid residues B_{N} and B_{C} selected from RBP with the amino acid sequence of the variable region of the cyclic peptide mP6-9 having an arbitrary linker amino acid added as needed to both sides of the amino acid sequence. The amino acid sequence of the loop portion, after fusion of the cyclic peptide, of the designed fusion protein is shown in FIG. 22.

### 2. Preparation of cyclic peptide fusion protein

A human RBP expression vector was prepared by linking DNA encoding the full-length RBP and DNA encoding a PA tag to each other and the resulting vector was incorporated in an expression vector pcDNA3.1 (product of Thermo Fisher Scientific). An expression vector was prepared by using the above-described vector and replacing the amino acid sequence of the loop region of L1 and L2 sites with the insertion sequence shown in FIG. 22. The insertion sequence portion was amplified by the extension PCR method to obtain a construct of various intended cyclic peptide fusion proteins. The cyclic peptide fusion proteins were called RBP(name of cyclic peptide_name of site), for example, RBP(mP6-9_L1).

SDS-PAGE analysis of RBP and two cyclic peptide fusion proteins (one peptide × two variations of inserted site) thereof was performed by carrying out transient expression using Expi293F cells by the method described in Example 1 and causing precipitation with Sepharose (product of Wako Pure Chemical Industries) having immobilized thereon NZ1, that is, an antibody against a PA tag from the culture supernatant.

The results of electrophoresis are shown in FIG. 23A. The band of RBP (Sample No. 1) was found at an expected molecular weight (25 kDa) and the band of the two cyclic peptide fusion proteins (Samples Nos. 2 and 3) appeared as a band showing mobility corresponding to a molecular weight slightly higher than that of the above band.

### 3. Preparation of cyclic peptide fusion protein and binding thereof to binding molecule

Binding of the resulting cyclic peptide fusion protein (RBP mutant) to Protein A Sepharose that had captured a soluble receptor fragment Plexin B1-Fc therein by a method similar to that described above was studied by the pulldown method.

The results of electrophoresis are shown in FIG. 23B. The pulldown results have revealed that wild type RBP (Sample No. 1) does not bind to Plexin B1-Fc, but two fusion proteins (Samples Nos. 2 and 3) having, incorporated therein, the sequence of the Plexin binder mP6-9 specifically binds to it. In other words, it has been confirmed that as in the case of Fn10-Fc, Fc, HSA, and hGH, the cyclic peptide can be converted into a fusion protein while retaining its binding activity to the binding molecule even by directly inserting it into the loop of the lipid-binding secretory protein RBP.

### [Example 7] Fusion between human placental alkaline phosphatase and cyclic peptide

### 1. Design of cyclic peptide fusion protein

In order to replace with the chemically crosslinked structure of a physiologically active cyclic peptide, human placental alkaline phosphatase (which will hereinafter be called "PLAP") was selected as an enzyme protein having a loop structure and serving as a scaffold protein. From the loop portion separated from the enzyme active site on the steric structure of the alkaline phosphatase, two amino acid residues Lys404 (corresponding to B_{N}) and Gly406 (corresponding to B_{C}) having a Cα atomic distance of 5.5 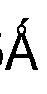 as a linking residue. The steric structure of the above portions is shown in FIG. 24.

A fusion protein was designed by replacing a portion of a region sandwiched between two amino acid residues B_{N} and B_{C} selected from PLAP with the amino acid sequence of the variable region of the cyclic peptide mP6-9 or aMD4 having an arbitrary linker amino acid added as needed to both sides of the amino acid sequence. The amino acid sequence of the loop portion, after fusion of the cyclic peptide, of the designed fusion protein is shown in FIG. 25.

### 2. Preparation of cyclic peptide fusion protein

As a human placental alkaline phosphatase expression vector, pAPtag-5 vector (described in Methods Enzymol. 2000, 327, 19-35) obtained by adding a Myc tag and a Hisx6 tag to the C terminal of the full-length PLAP was used. An expression vector was prepared by using the above-described vector and replacing the loop region with the insertion sequence shown in FIG. 25. The insertion sequence portion was amplified by the extension PCR method to obtain a construct of various intended cyclic peptide fusion proteins. The cyclic peptide fusion proteins were each called PLAP(name of cyclic peptide), for example, PLAP(mP6-9).

SDS-PAGE analysis of PLAP and two cyclic peptide fusion proteins (two peptides × one inserted site) thereof was performed by carrying out transient expression using Expi293F cells by the method described above in Example 1 and causing precipitation with Ni-NTA agarose from the culture supernatant.

The results of electrophoresis are shown in FIG. 26. The band of PLAP (Sample No. 1) was found at an expected molecular weight (74 kDa) and the band of the two cyclic peptide fusion proteins (Samples Nos. 2 and 3) appeared as a band showing mobility corresponding to a molecular weight slightly higher than that of the above band.

### 3. Binding of cyclic peptide fusion protein to binding molecule

Binding of the resulting cyclic peptide fusion protein (PLAP mutant) to Protein A Sepharose that had captured a soluble receptor fragment Plexin B1-Fc or Met-Fc therein by the method described above I Example 1 was studied by the pulldown method.

The results of electrophoresis are shown in FIG. 27. The pulldown results have revealed that wild type PLAP (Sample No. 1) does not bind to Plexin B1-Fc or Met-Fc, but the fusion protein (Sample No. 2) having, incorporated therein, the sequence of the Plexin binder mP6-9 and the fusion protein (Sample No. 3) having, incorporated therein, the sequence of aMD4 specifically bind to PlexinB1-Fc and Met-Fc, respectively. In other words, it has been confirmed that as in the case of Fn10-Fc, Fc, HSA, hGH, and RBP, the cyclic peptide can be converted into a fusion protein while retaining its binding activity to the binding molecule even by directly inserting it into the loop of the enzyme protein PLAP.

### [Example 8] Physiological activity of cyclic protein fusion peptide

The Plexin B1 binder peptide P6 and analog thereof mP6-9 bind to Plexin B1 expressed on cells and inhibit its signal transduction. In order to study whether or not a peptide fused to a protein exhibits this inhibiting activity, an influence of the stimulation of Semaphorin 4D (Sema4D) on a morphological change of Plexin B1 expression cells was evaluated.

The morphological change of the cells was measured by the impedance method using a xCELLigence RTCA DP apparatus (product of ACEA Biosciences). After Plexin B1 stable expression cells (described in Cell Chem. Biol, 2016, 23, 1341-1350) were seeded on an E-Plate View 16 PET plate (product of ACEA Biosciences) at a concentration of 6,000 cells/well, they were cultured at 37°C for 20 hours. Then, 1 nM to 1 µM of the mP6-9 peptide fusion protein described in Example 2 was added to the medium and kept warm for 30 minutes. As a stimulant, a Sema4D-Fc protein (product of R&D Systems) was added to give a final concentration of 1 nM and the impedance of the cells was monitored.

The results are shown in FIG. 28. Immediately after addition of Sema4D-Fc, a drastic reduction in impedance occurred, suggesting that a morphological change of cells occurred and an adhesion area retracted. It has been revealed, on the other hand that the morphological change of cells pretreated with the six fusion proteins were inhibited depending on the concentration of the fusion protein added and all the fusion proteins blocked the activation of Plexin B1 almost completely at 100 nM or more. This means that the original efficacy of the cyclic peptide mP6-9 as a Plexin B1 signal transduction inhibitor is maintained even after conversion into a fusion protein.

## Claims

1. A method of presenting an amino acid sequence derived from a cyclic peptide, having a binding activity to an intended molecule, on a scaffold protein having a loop structure, the method comprising:
using, as the cyclic peptide, a peptide having a chemically crosslinked structure thereby forming an intramolecular cyclic structure, and
replacing the chemically crosslinked structure of the cyclic peptide with two amino acid residues from the loop structure of the scaffold protein and fusing the resulting amino acid sequence derived from the cyclic peptide to the scaffold protein while retaining the binding activity of the cyclic peptide to the intended molecule, wherein the two amino acid residues from the loop structure have a Cα atomic distance within a range of from 4 to 7Ǻ.

2. The method according to Claim 1, wherein the scaffold protein having a loop structure has from 1 to 15 amino acid residues between the two amino acid residues from the loop structure.

3. The method according to Claim 1 or 2, wherein the cyclic peptide is composed of a proteinogenic amino acid and/or a non-proteinogenic amino acid.

4. The method according to any one of Claims 1 to 3, wherein the chemically crosslinked structure contains a thioether bond or a disulfide bond.

5. The method according to any one of Claims 1 to 4, wherein two or more cyclic peptides each fuse to respectively different loop structures of a scaffold protein having a plurality of loop structures.

6. The method according to Claim 5, wherein the two or more cyclic peptides have the same amino acid sequence.

7. The method according to Claim 5, wherein the two or more cyclic peptides have respectively different amino acid sequences.

8. The method according to any one of Claims 1 to 7, wherein in replacing the chemically crosslinked structure of the cyclic peptide with the two amino acid residues from the loop structure, the chemically crosslinked structure of the cyclic peptide is replaced, via a linker sequence, with the two amino acid residues from the loop structure.

9. The method according to any one of Claims 1 to 8, wherein the chemically crosslinked structure of the cyclic peptide is replaced with the two amino acid residues from the loop structure while including an amino acid sequence constituting the loop structure other than the two amino acids from the loop structure.

10. A method of fusing a partial amino acid sequence derived from a cyclic peptide, having a binding activity to an intended molecule, to a scaffold protein having a loop structure, thereby producing a modified protein having the partial amino acid sequence derived from the cyclic peptide presented on the protein, the method comprising:
using, as the cyclic peptide, a peptide having a chemically crosslinked structure thereby forming an intramolecular cyclic structure,
selecting, from an amino acid sequence of the cyclic peptide, a partial amino acid sequence to be presented on the scaffold protein and selecting a nucleobase sequence corresponding to the partial amino acid sequence,
selecting nucleobase sequences corresponding to two amino acid sequences from the loop structure of the scaffold protein, respectively, removing a base present between the selected nucleobase sequences if necessary, and preparing a nucleic acid having, inserted and incorporated therein, the nucleobase sequence corresponding to the partial amino acid sequence of the selected cyclic peptide, and
translating the nucleic acid wherein the resulting modified protein retains the binding activity to the intended molecule,
wherein the two amino acid residues constituting the loop structure have a Cα atomic distance within a range of from 4 to 7Ǻ.

## Patentansprüche

1. Verfahren zum Präsentieren einer Aminosäuresequenz, die aus einem cyclischen Peptid mit einer Bindungsaktivität für ein vorgesehenes Molekül stammt, auf einem Gerüstprotein mit einer Schleifenstruktur, wobei das Verfahren Folgendes umfasst:
Bilden einer intramolekularen cyclischen Struktur unter Verwendung eines Peptids mit einer chemisch vernetzten Struktur als cyclisches Peptid und
Ersetzen der chemisch vernetzten Struktur des cyclischen Peptids durch zwei Aminosäurereste aus der Schleifenstruktur des Gerüstproteins und Fusionieren der aus dem cyclischen Peptid stammenden resultierenden Aminosäuresequenz an das Gerüstprotein unter Beibehaltung der Bindungsaktivität des cyclischen Peptids für das vorgesehene Molekül, wobei die zwei Aminosäurereste aus der Schleifenstruktur einen Cα-Atomabstand im Bereich von 4 bis 7 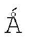 aufweisen.

2. Verfahren nach Anspruch 1, wobei das Gerüstprotein mit einer Schleifenstruktur 1 bis 15 Aminosäurereste zwischen den zwei Aminosäureresten aus der Schleifenstruktur aufweist.

3. Verfahren nach Anspruch 1 oder 2, wobei das cyclische Peptid aus einer proteinogenen Aminosäure und/oder einer nicht proteinogenen Aminosäure besteht.

4. Verfahren nach einem der Ansprüche 1 bis 3, wobei die chemisch vernetzte Struktur eine Thioetherbindung oder eine Disulfidbindung enthält.

5. Verfahren nach einem der Ansprüche 1 bis 4, wobei zwei oder mehr cyclische Peptide jeweils an verschiedene Schleifenstrukturen eines Gerüstproteins mit mehreren Schleifenstrukturen fusionieren.

6. Verfahren nach Anspruch 5, wobei die zwei oder mehr cyclischen Peptide die gleiche Aminosäuresequenz aufweisen.

7. Verfahren nach Anspruch 5, wobei die zwei oder mehr cyclischen Peptide jeweils verschiedene Aminosäuresequenzen aufweisen.

8. Verfahren nach einem der Ansprüche 1 bis 7, wobei beim Ersetzen der chemisch vernetzten Struktur des cyclischen Peptids durch die zwei Aminosäurereste aus der Schleifenstruktur die chemisch vernetzte Struktur des cyclischen Peptids über eine Linkersequenz durch die zwei Aminosäurereste aus der Schleifenstruktur ersetzt wird.

9. Verfahren nach einem der Ansprüche 1 bis 8, wobei die chemisch vernetzte Struktur des cyclischen Peptids durch die zwei Aminosäurereste aus der Schleifenstruktur unter Einschluss einer die Schleifenstruktur aufbauenden Aminosäuresequenz, die von den zwei Aminosäuren aus der Schleifenstruktur verschieden ist, ersetzt wird.

10. Verfahren zum Fusionieren einer aus einem cyclischen Peptid stammenden Teil-Aminosäuresequenz mit einer Bindungsaktivität für ein vorgesehenes Molekül an ein Gerüstprotein mit einer Schleifenstruktur, wodurch ein modifiziertes Protein mit der aus dem cyclischen Peptid stammenden auf dem Protein präsentierten Teil-Aminosäuresequenz erzeugt wird, wobei das Verfahren Folgendes umfasst:
Bilden einer intramolekularen cyclischen Struktur unter Verwendung eines Peptids mit einer chemisch vernetzten Struktur als cyclisches Peptid,
Auswählen einer auf dem Gerüstprotein zu präsentierenden Teil-Aminosäuresequenz aus einer Aminosäuresequenz des cyclischen Peptids und Auswählen einer Nukleobasensequenz, die der Teil-Aminosäuresequenz entspricht,
Auswählen von Nukleobasensequenzen, die jeweils zwei Aminosäuresequenzen aus der Schleifenstruktur des Gerüstproteins entsprechen, Entfernen einer zwischen den ausgewählten Nukleobasensequenzen vorhandenen Base,
sofern notwendig, und Herstellen einer Nukleinsäure mit der darin eingeführten und eingebauten Nukleobasensequenz, die der Teil-Aminosäuresequenz des ausgewählten cyclischen Peptids entspricht, und
Translatieren der Nukleinsäure, wobei das resultierende modifizierte Protein die Bindungsaktivität für das vorgesehene Molekül beibehält,
wobei die zwei die Schleifenstruktur aufbauenden Aminosäurereste einen Cα-Atomabstand im Bereich von 4 bis 7 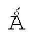 aufweisen.

## Revendications

1. Procédé de présentation d'une séquence d'acides aminés dérivée d'un peptide cyclique, possédant une activité de liaison à une molécule cible, sur une protéine d'échafaudage ayant une structure en boucle, le procédé comprenant :
l'utilisation, en tant que peptide cyclique, d'un peptide possédant une structure chimiquement réticulée, formant ainsi une structure cyclique intramoléculaire, et
le remplacement de la structure chimiquement réticulée du peptide cyclique par deux résidus d'acides aminés provenant de la structure en boucle de la protéine d'échafaudage, et la fusion de la séquence d'acides aminés ainsi obtenue, dérivée du peptide cyclique, avec la protéine d'échafaudage, tout en conservant l'activité de liaison du peptide cyclique à la molécule cible, les deux résidus d'acides aminés provenant de la structure en boucle présentant une distance atomique Cα comprise entre 4 et 7 Å.

2. Procédé selon la revendication 1, dans lequel la protéine d'échafaudage ayant une structure en boucle comporte 1 à 15 résidus d'acides aminés entre les deux résidus d'acides aminés provenant de la structure en boucle.

3. Procédé selon la revendication 1 ou 2, dans lequel le peptide cyclique est composé d'un acide aminé protéinogène et/ou d'un acide aminé non protéinogène.

4. Procédé selon l'une quelconque des revendications 1 à 3, dans lequel la structure chimiquement réticulée contient une liaison thioéther ou une liaison disulfure.

5. Procédé selon l'une quelconque des revendications 1 à 4, dans lequel au moins deux peptides cycliques fusionnent chacun avec des structures en boucle différentes d'une protéine d'échafaudage comportant plusieurs structures en boucle.

6. Procédé selon la revendication 5, dans lequel les au moins deux peptides cycliques ont la même séquence d'acides aminés.

7. Procédé selon la revendication 5, dans lequel les au moins deux peptides cycliques ont des séquences d'acides aminés différentes.

8. Procédé selon l'une quelconque des revendications 1 à 7, dans lequel, lors du remplacement de la structure chimiquement réticulée du peptide cyclique par les deux résidus d'acides aminés provenant de la structure en boucle, la structure chimiquement réticulée du peptide cyclique est remplacée, via une séquence de lieur, par les deux résidus d'acides aminés provenant de la structure en boucle.

9. Procédé selon l'une quelconque des revendications 1 à 8, dans lequel la structure chimiquement réticulée du peptide cyclique est remplacée par les deux résidus d'acides aminés provenant de la structure en boucle tout en incluant une séquence d'acides aminés constituant la structure en boucle autre que les deux acides aminés provenant de la structure en boucle.

10. Procédé de fusion d'une séquence d'acides aminés partielle dérivée d'un peptide cyclique, possédant une activité de liaison à une molécule cible, avec une protéine d'échafaudage ayant une structure en boucle, produisant ainsi une protéine modifiée sur laquelle la séquence d'acides aminés partielle dérivée du peptide cyclique est présentée, le procédé comprenant :
l'utilisation, en tant que peptide cyclique, d'un peptide possédant une structure chimiquement réticulée, formant ainsi une structure cyclique intramoléculaire,
la sélection, à partir d'une séquence d'acides aminés du peptide cyclique, d'une séquence d'acides aminés partielle à présenter sur la protéine d'échafaudage et
la sélection d'une séquence de nucléobases correspondant à la séquence d'acides aminés partielle,
la sélection de séquences de nucléobases correspondant à deux séquences d'acides aminés de la structure en boucle de la protéine d'échafaudage, respectivement,
l'élimination d'une base présente entre les séquences de nucléobases sélectionnées, si nécessaire, et la préparation d'un acide nucléique dans lequel est insérée et incorporée la séquence de nucléobases correspondant à la séquence d'acides aminés partielle du peptide cyclique sélectionné, et
la traduction de l'acide nucléique, la protéine modifiée résultante conservant l'activité de liaison à la molécule cible,
les deux résidus d'acides aminés constituant la structure en boucle présentant une distance atomique Cα comprise entre 4 et 7 Å.
